# EUROPEAN PATENT SPECIFICATION

(11) **EP 3 823 567 B1**
(45) Date of publication and mention of the grant of the patent: **18.06.2025**
(21) Application number: 19756444.6
(22) Date of filing: 18.07.2019
(51) Int. Cl.: A61N 1/30, A61F 9/00, A61N 1/32, A61K 9/00, A61K 48/00, A61N 1/36, C12N 15/87

(54) **INTRAOCULAR INJECTION OF GENE THERAPY EXPRESSION VECTORS**
INTRAOKULARE INJEKTION VON EXPRESSIONSVEKTOREN FÜR GENTHERAPIE
INJECTION INTRAOCULAIRE DE VECTEURS D'EXPRESSION DE THÉRAPIE GÉNIQUE

(30) Priority: 20.07.2018 US 201862701267 P; 29.08.2018 US 201862724480 P; 16.11.2018 US 201862768590 P
(43) Date of publication of application: 26.05.2021
(73) Proprietor: The USA, as represented by The Secretary, Department of Health and Human Services, Bethesda, MD 20892-7788 (US)
(72) Inventor: SIEVING, Paul Albert, Bethesda, Maryland 20817 (US); BUSH, Ronald Avery, Howell, Michigan 48843 (US); SONG, Hongman, Rockville, Maryland 20852 (US); WEI, Lisa L., Gaithersburg, Maryland 20878 (US); ZENG, Yong, Potomac, Maryland 20854 (US); WILEY, Henry Ernest, Bethesda, Maryland 20817 (US)
(74) Representative: Titmus, Craig Edward
(86) International application number: PCT/US2019/042365
(87) International publication number: WO 2020/018766

(56) References cited:
- US-A1- 2009 088 721
- US-A1- 2018 066 022
- CUKRAS CATHERINE ET AL: "Retinal AAV8-RS1 Gene Therapy for X-Linked Retinoschisis: Initial Findings from a Phase I/IIa Trial by Intravitreal Delivery", vol. 26, no. 9, 7 July 2018 (2018-07-07), pages 2282 - 2294, XP009511982, ISSN: 1525-0016, Retrieved from the Internet <URL:https://www.sciencedirect.com/science/article/pii/S1525001618302582?via%3Dihub> DOI: 10.1016/J.YMTHE.2018.05.025
- ISHIKAWA H ET AL: "Effect of GDNF gene transfer into axotomized retinal ganglion cells using in vivo electroporation with a contact lens-type electrode", GENE THERAPY, NATURE PUBLISHING GROUP, LONDON, GB, vol. 12, no. 4, February 2005 (2005-02-01), pages 289 - 298, XP008130639, ISSN: 0969-7128, [retrieved on 20041223], DOI: 10.1038/SJ.GT.3302277
- HONGMAN SONG ET AL: "Trans-ocular Electric Current In Vivo Enhances AAV-Mediated Retinal Gene Transduction after Intravitreal Vector Administration", MOLECULAR THERAPY - METHODS & CLINICAL DEVELOP, vol. 13, June 2019 (2019-06-01), GB, pages 77 - 85, XP055634872, ISSN: 2329-0501, DOI: 10.1016/j.omtm.2018.12.006

## Description

### TECHNICAL FIELD

The present invention relates to the treatment of disorders of the eye. In particular, the invention relates to viral expression vectors capable of modulating a target gene or gene product for the treatment of disorders of the eye.

### BACKGROUND

Recombinant vectors (for example, adeno-associated viral (AAV) vectors) show promise for ocular gene therapy, with positive safety and efficacy results demonstrated in preclinical studies and clinical trials. Successful clinical application of AAV-based gene therapies requires efficient transduction and expression by target cells. As many inherited retinal degenerations involve genes expressed in photoreceptors and retinal pigment epithelium (RPE), these are important cells for therapeutic gene expression.

The route of vector administration affects the efficacy of retinal gene therapy. Commonly used AAV serotypes 1, 2, 5, 8, 9 and rh10 efficiently transduce the RPE and/or photoreceptors in the wild-type (WT) adult rodent retina only when given by subretinal injection. Unfortunately, this limits vector distribution primarily to the region surrounding the site of subretinal administration. This approach may also cause injury, particularly for degenerative and surgically vulnerable retinal conditions. Intravitreal injection is more desirable as a less invasive way to deliver AAV vectors to the retina. Following intravitreal injection, AAV vectors diffuse through the vitreous humor and distribution theoretically reaches the entire retina. However, the extensive laminated structure of the vertebrate retina further limits AAV vectors from reaching cells in outer retina after vitreous application, and these RPE and photoreceptor cells often show limited transduction. Approaches to overcome such tissue barriers (e.g. diffusion and membranes) include mild enzymatic digestion of the inner limiting membrane (ILM), vitrectomy, and surgical ILM peeling. Although enhanced viral transduction of multiple retinal cell types is sometimes observed using these approaches, a more effective, efficient, and convenient method of administering gene therapy vectors to the eye is needed.

Methods for treatment of the eyes are already known from the following publications:
- ISHIKAWA H ET AL: "Effect of GDNF gene transfer into axotomized retinal ganglion cells using in vivo electroporation with a contact lens-type electrode", GENE THERAPY, NATURE PUBLISHING GROUP, LONDON, GB, vol. 12, no. 4, February 2005, pages 289-298, ISSN: 0969-7128, DOI: 10.1038/SJ.GT.3302277
- US 2009/088721 A1 (DE BIZEMONT THERESE [FR] ET AL) 2 April 2009
- US 2018/066022 A1 (CHALBERG THOMAS W [US] ET AL) 8 March 2018
- CUKRAS CATHERINE ET AL: "Retinal AAV8-RS1 Gene Therapy for X-Linked Retinoschisis: Initial Findings from a Phase I/IIa Trial by Intravitreal Delivery", MOLECULAR THERAPY, ELSEVIER INC, US, vol. 26, no. 9 7 July 2018, pages 2282-2294, XP009511982, ISSN: 1525-0016, DOI: 10.1016/J.YMTHE.2018.05.025, URL:https://www.sciencedirect.com/science/ article/pii/S1525001618302582?via%3Dihub

### SUMMARY

The inventors have explored strategic intravitreal placement of gene therapy vectors alone and in combination with the use of low electric current stimulation applied across the eye *in vivo* to enhance delivery of gene therapy vectors, including therapeutic adeno-associated viral (AAV) vector constructs administered into the vitreous. AAV is an icosahedral non-enveloped DNA virus that is relatively thermostable and resistant to mild proteolytic digestion and nonionic detergents. It has an overall net negative charge in a neutral environment and is therefore a model gene therapy vector for testing intraocular administration and gene therapy expression studies. Using these models, the inventors have developed and tested a novel, non-invasive approach of applying electric current (electric-current vector mobility [ECVM]) with intravitreal vector injection and showed that this strategy significantly improves the transduction efficiency of AAV vectors in wild type (WT) mouse retina.

Thus, the present invention is is defined by the appended claims. Methods disclosed hereinafter are not specifically claimed but are useful for understanding the present invention.

The present disclosure presents exemplary methods for the treatment of a disease or disorder of the eye comprising administering to a subject an expression vector capable of modulating a target gene or gene product in a therapeutically effective amount by administering the expression vector by intravitreal injection alone or in conjunction with the application of an electric current to the injected eye.

In these methods, the gene therapy vector may be any suitable expression vector for effecting gene therapy in the eye of a mammal. This may include, for example, retroviruses, adenoviruses, and/or neurotropic viruses. These administration methods may be applied to encapsulated vectors (e.g., nanoparticles), modified DNA or vector (e.g., pegylation as a modification), and may also be applied to the administration of naked DNA plasmids to the eye.

These methods enhance the delivery of gene therapy vectors into and through tissues, e.g., tissues of the eye. More specifically, the methods described herein utilize the application of an electric current to actively deliver a gene therapy expression vector, e.g., an AAV vector, into a mammalian eye. The methods focus on the effective delivery of expression vectors to maximize gene delivery, protein expression, and distribution in the eye, and in particular beyond the inner limiting membrane (ILM) of the eye, and within the photoreceptors, bipolar cells, amacrine cells, retinal ganglion cells, and/or retinal pigment epithelia (RPE).

The methods encompass different electric current levels and application times to optimize delivery, expression, and distribution of the applied ocular gene therapy. These administration methods can lead to results comparable to, or better than, those achieved by subretinal injection alone, without the significant risk of infection or retinal trauma.

For example, in gene therapy treatment of X-linked juvenile retinoschisis (XLRS), the target for the gene delivery is the target cells for protein expression, the photoreceptors of the eye, located behind other cells of the retina. The photoreceptors are essentially nerve cells, some of which are in contact with retinal ganglion cells. The retinal ganglial cells are bundled and have a long, extending structure that makes up the optic nerve extending into the brain. Retinal ganglia are very sensitive to insults of disease and injury. Thus, the ability to deliver gene therapies to these cells by administration method(s) that prevents or substantially reduces injury or death of these cells represents a significant increase in safety and efficacy in ocular gene therapy directed to these cells for ocular diseases such as XLRS.

This disclosure is directed to exemplary methods for delivering a gene therapy vector into the eye of a subject, by administering the gene therapy vector to the eye of the subject by injection, and applying an electric current to the injected eye, thereby delivering the gene therapy vector into the eye or into the retina.

In these methods, the method of injection may be selected from the group consisting of an intracameral injection, an intracorneal injection, a subconjunctival injection, a subtenon injection, a subretinal injection, an intravitreal injection, a suprachoroidal injection, and an injection in the anterior chamber of the eye. Preferably, the injection is an intravitreal injection.

As used in this disclosure, "para-retinal" intravitreous injection of a gene vector (or other therapeutic agent) involves targeted delivery by injection of the vector into the vitreous cavity in close proximity to the desired region of the retina (for example, near the fovea area of the retina). In contrast to routine intravitreous injections, which are done using short needles designed to deposit product in the mid-vitreous cavity, and do not require direct visualization, para-retinal injection is done under direct visualization of a longer needle capable of delivering product in the posterior vitreous cavity close to the retina. The therapeutic agent (e.g., the gene therapy vector) may be deposited in the vitreous cavity at a distance of 0mm to 13mm from the retina, or at a distance of 0mm to 10mm from the retina, at a distance of 0mm to 5mm from the retina, or preferably at a distance of 0mm to 3mm from the retina. One version of this delivery technique involves the use of a small gauge needle (30 gauge or similar) with length sufficient to reach the posterior pole of the human eye (25 mm or similar), exo- or endo-illumination and visualization using a microscope, and use of a corneal contact lens to allow focus on the posterior vitreous cavity and retina. This is typically conducted after adequate analgesia and antisepsis, at which time the corneal contact lens is coupled to the eye and the microscope is positioned to view the posterior retina. The needle is inserted through the eye wall in the pars plana region and its tip is visualized. Under direct visualization, the needle tip is advanced to the desired location close to the retinal surface. The syringe plunger is advanced to slowly deposit the vector (which may be contained in any suitable composition or formulation). The needle is withdrawn and the eye is inspected. The port may be closed with a suture, but for a sufficiently small-caliber needle (such as 30 gauge), no suture is needed to close the needle tract. Ointment and an eye shield may be applied and, if desired, the subject can be kept in a supine position for a period postoperatively to further facilitate a high para-retinal concentration of the product. Variations on delivery instrumentation can include creation of a sclerotomy with or without the use of a vitrectomy port to allow use of a blunt cannula, and/or a cannula design with a tapered and/or flexible extendable tip or side ports to optimize proximity to the retinal surface and safety, and/or use of a pneumatic system in lieu of a simple syringe plunger.

Without intending to be bound by theory, the electric current applied to the subject following the intraocular injection of the expression vector may impart a substantially enhanced delivery of the vectors generally by the movement of charged particles within the electric field established by the application of the current. The effective mechanism may also be the disruption of biological membrane barriers, or tissues or cell layers, such as retinal tissue layers, and therefore the administration methods of this disclosure may be effective for significantly enhancing the delivery of charged as well as uncharged expression vectors. Alternatively or additionally, the application of the electric current may cause the synthesis and/or release of trophic or other factors that improve the delivery of the expression vector. For example, the application of the electric current may cause enhanced expression of trophic factors such as basic fibroblast growth factor (bFGF), which may ultimately enhance the expression of gene therapy vectors in the tissues of the eye. Alternatively or additionally, the electric current may alter the charge present across the cell layers of the tissue. Alternatively or additionally, the electric current may augment the uptake of expression vectors thereby increasing cell expression. Thus, the electric current may provide a stimulus that may be electromotivate, electroconduct, electroinduct, electrotransport, and/or electrophorese one or more therapeutic materials into areas of target tissue in the eye, thereby placing one or more therapeutic biologic or other materials in proximity to, on, or within target tissue(s) in the eye.

In these methods, the ocular administration in conjunction with the application of an electric current may be carried out prior to, during, or after the step of injecting the gene therapy vector.

In these methods, the delivered gene therapy expression vector may be virus or a viral expression vector. In examples herein where the expression vector is a virus, the virus can be an adenovirus, an adeno-associated virus (AAV), a retrovirus, vaccinia virus, herpes simplex virus, a lentivirus, a baculovirus, a recombinant virus that contains a nucleic acid molecule that is heterologous to its genome, a replication-defective virus, a virus that replicates in the nucleus of a cell in the subject, a virus that infects non-dividing cells or dividing cells, a virus that exhibits tropism for tissues of the eye. In exemplary embodiments, the gene therapy vector may be an adeno-associated viral (AAV) vector or derivative thereof, comprising a nucleic acid sequence encoding a therapeutic protein, wherein the expression vector expresses a high level of the therapeutic protein. The nucleic acid encoding the therapeutic protein may be linked to an eye-specific promoter. Further, the promoter may be specific for certain tissues of the eye, such as the retina. In such embodiments, a retina-specific promoter may comprise a portion of a retinoschisin gene promoter. In these methods, the expression vector may be associated with a nanoparticle, which may be a targeted or radiolabeled nanoparticle.

In these methods, the gene therapy vector is delivered in conjunction with an electric current of 1.0 micro-amperes (µA) or greater applied for a duration of seconds, minutes, or hours. In these methods, the electric current may be applied at a variable or fixed current (i.e., a steady or pulsed current), or using a biphasic current (i.e., periodically flipping the polarity between negative and positive). The electric current may be applied for a period of seconds, such as 1-60 seconds. The electric current may be applied for a period of minutes, such as 1-60 minutes. The electric current may be applied for a period of hours, such as 1-20 hours. The electric current may be applied over a period of seconds, minutes, or hours, in a single application or as separate, repeated applications, such as on separate days.

In these methods, the disease or disorder of the eye to be treated by the administration of the gene therapy in conjunction with the application of an electric current to the eye may be any monogenic or polygenic retinal disease including, for example, a disease or disorder selected from Stargardt disease, retinoschisis, age-related macular degeneration (AMD), diabetic retinopathy, Leber congenital amaurosis (LCA), retinal detachment (due to disease, injury or spontaneous detachment), cysts, cystoid macular edema, retinitis pigmentosa, senile schisis, Fuchs corneal dystrophy and other corneal dystrophies, glaucoma, and cataract. In a preferred embodiment, the disease of the eye is X-linked juvenile retinoschisis (XLRS).

Another aspect of this disclosure is a kit for delivering gene therapy vectors into the eye of a subject, wherein the kit is to be used for applying an electric current of about 1.0 micro-amperes (µA) or greater, for a time period of seconds to hours, at a steady or intermittent current. These kits may include components of an apparatus, for delivering an expression vector, or nanoparticles comprising an expression vector, into the eye of a subject. Optionally, these kits may include one or more gene therapy vectors suitable for delivery of a gene therapy construct encoding a therapeutic protein to the eye of a subject.

The present invention is defined by the appended claims.

Additional aspects of the present invention will become more readily apparent from the Detailed Description, particularly when taken together with the figures.

### BRIEF DESCRIPTION OF THE FIGURES

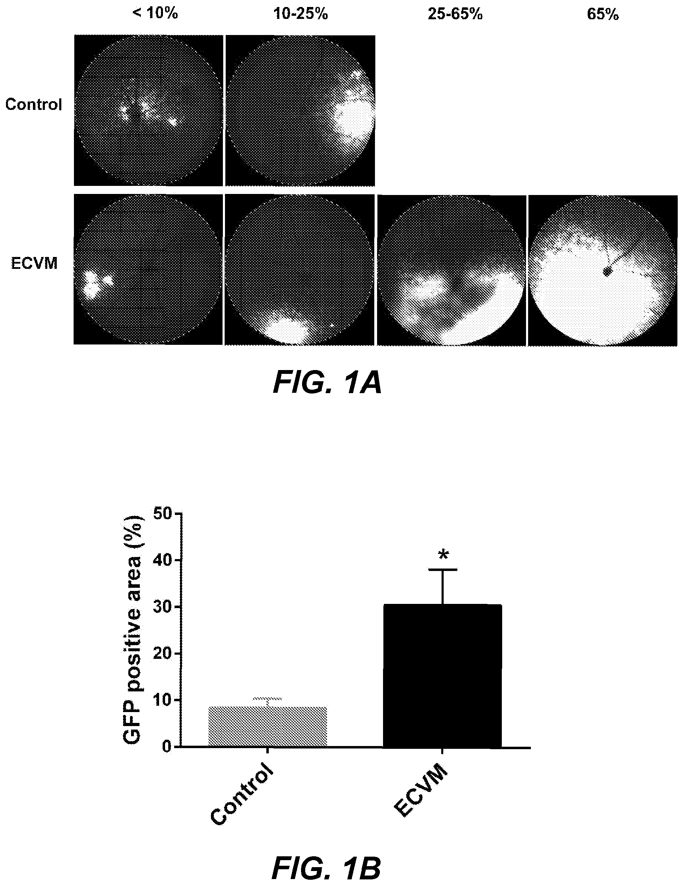

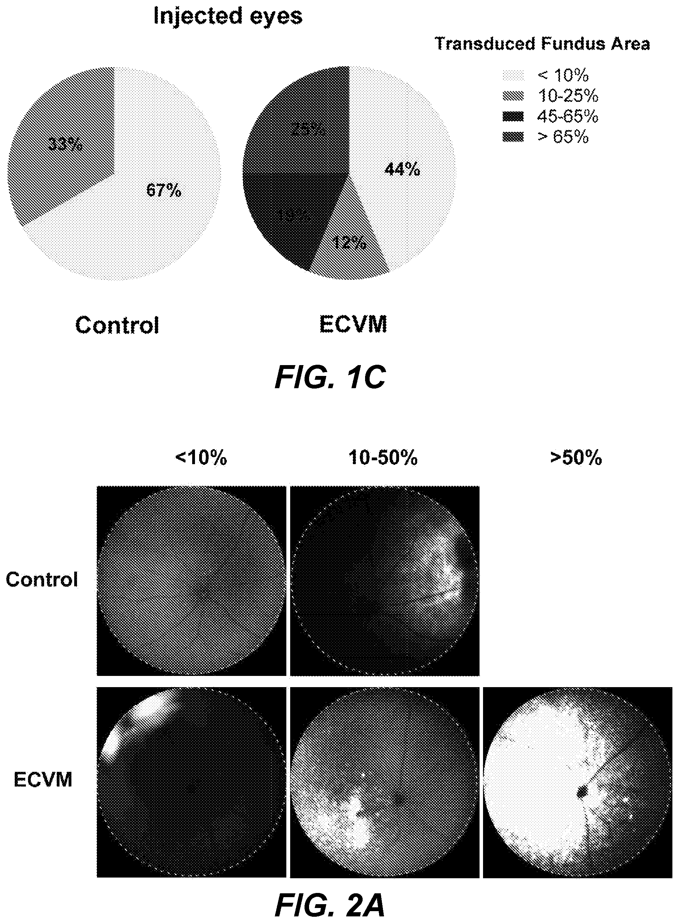

**FIGS. 1A-1C** show that the application of electric-current vector mobility (ECVM) significantly enhances AAV8-mediated gene transduction in wild-type (WT) retinas following intravitreal delivery. WT mouse eyes were treated with ECVM (continuous direct current, 10µA for 20 min) immediately after intravitreal injection of AAV8-CMV-EGFP. Vector-injected WT eyes without applying ECVM served as controls. Fundus images were taken at 6 weeks post-injection (PI). **FIG. 1A** shows a representative fundus image of injected-ECVM treated eyes (ECVM) and injected eyes without applying current (Control) are shown from samples with different GFP expression (<10%, 10-25%, 25-65%, >65%) in terms of fundus area transduced. Dashed circle outlines the central retina. **FIG.** 1B shows GFP-positive areas of fundus images quantified and represented as an average percentage of the fundus area showing GFP expression (mean ± SEM) for control (n = 21) and ECVM (n = 16) groups, respectively. * p < 0.05, Mann Whitney test. **FIG. 1C** is pie graphs showing the probability of injected eyes having a GFP positive area coverage more than 65%, between 25% and 65%, between 10% and 25%, and less than 10%.

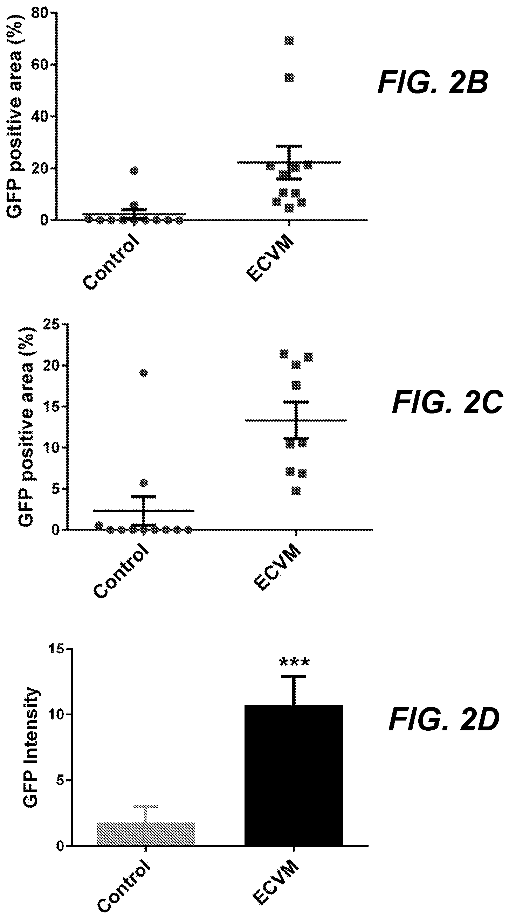

**FIGS. 2A-2D** show that the application of ECVM increases transduction efficacy of AAV vectors in WT retinas from the vitreous at a reduced vector dose. WT mouse eyes were treated with ECVM (continuous direct current, 10µA for 20 min) immediately after intravitreal delivery of AAV8-CMV-EGFP. Vector-injected WT eyes without ECVM application served as controls. Fundus images were taken at 4.5 weeks post-injection (PI). **FIG. 2A** shows representative fundus images of injected eyes with (ECVM) and without electric current (Control) were shown from samples with different GFP expression (<10%, 10-50%, >50%) in terms of fundus area transduced. Dashed circle outlines the central retina. **FIG. 2B** shows GFP positive area of the fundus image from each sample quantified and represented as a percentage of the fundus area showing GFP expression (p < 0.0001, Mann Whitney test, n = 11 for each group). **FIG. 2C** shows the difference between two groups was highly significant with two samples showing largest retinal area transduced (outliers) removed from ECVM group (p = 0.0003, Mann Whitney test, n=9 for ECVM group and n=11 for Control group). FIG. 2D shows GFP immunofluorescence of representative retinal sections was quantified and plotted as GFP intensity (pixel/area, mean ± SEM) for Control and ECVM groups, respectively. *** p = 0.0007, Mann Whitney test.

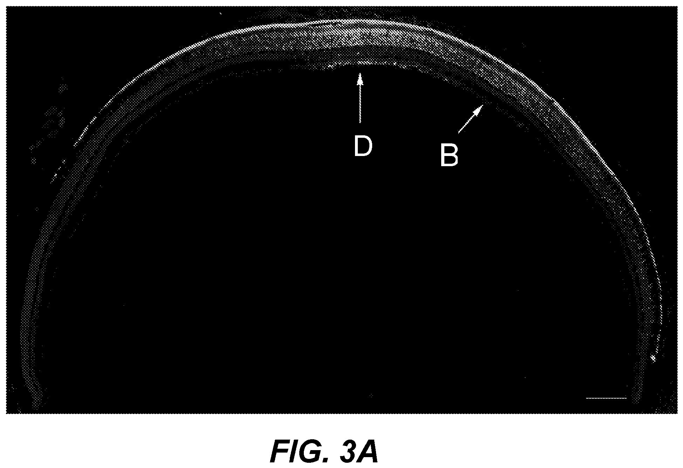

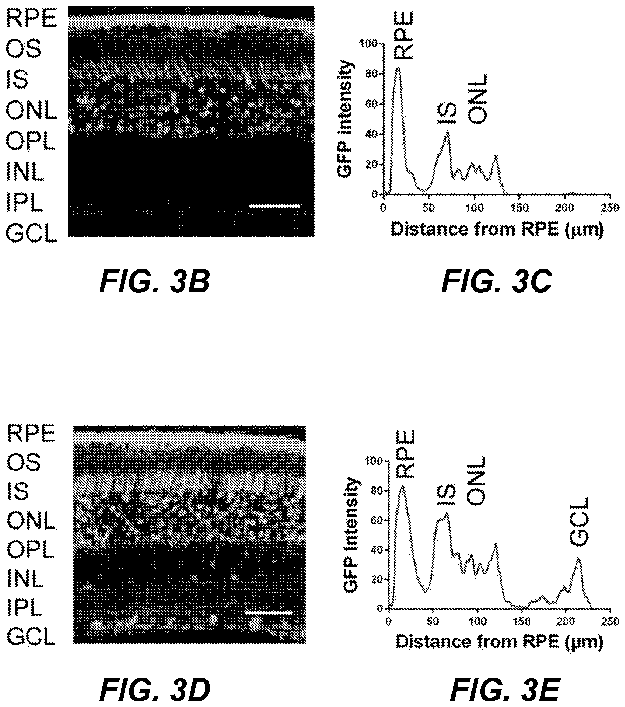

**FIGS. 3A-3E** show AAV8-GFP vectors penetrate all the retinal layers from the vitreous with ECVM application. WT mouse eyes were treated with ECVM (continuous direct current, 10µA for 20 min) immediately after intravitreal delivery of AAV8-CMV-EGFP (5E 8 vg/eye) and collected 5 weeks post injection. **FIG. 3A** is a representative large image of the unimmunostained retina in cross-section of vector injected ECVM treated eyes illustrates distribution of transduced cells in the retina, showing original GFP fluorescence throughout the retinal layers. At least three-quarters of the entire length of the RPE and ONL regions was transduced to some degree by the AAV8 GFP vector. *Scale bar:* 200 µm. Higher magnification images show florescence profiles across the retinal thickness (**FIGS. 3B-3E**). GFP signals were observed in RPE and ONL (photoreceptors) (**FIGS. 3B** and **3C**) or in nearly all retinal layers (**FIGS. 3D** and **3E**). RPE - retinal pigment epithelium, OS - outer segments, IS - inner segments, ONL - outer nuclear layer, OPL - outer plexiform layer, INL - inner nuclear layer, IPL - inner plexiform layer, GCL - ganglion cell layer. Scale bar: 50 µm.

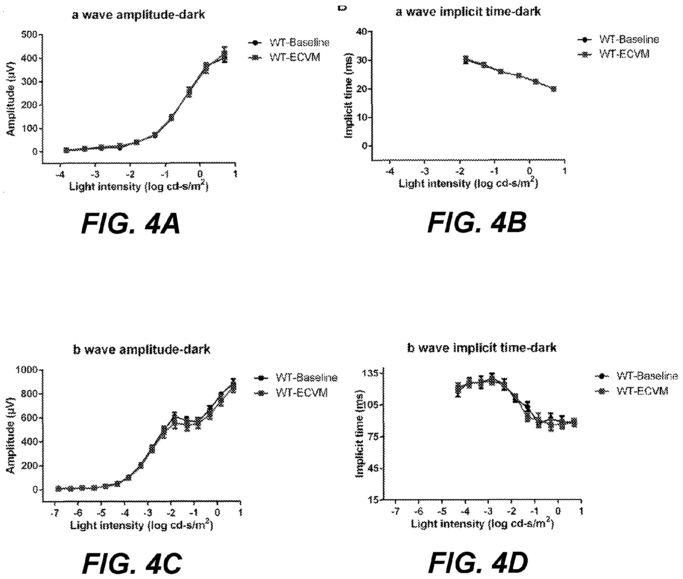

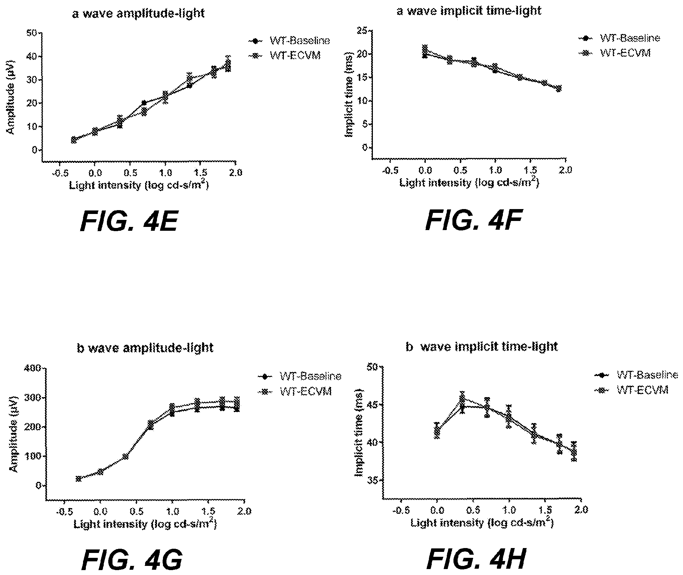

**FIGS. 4A-4H** show electric-current vector mobility (ECVM)-treated eyes have normal retinal function. Wild type mouse eyes (n=7) were treated with ECVM (monophasic, 10µA for 20 min). Retinal function of these mice was measured and compared by electroretinograms (ERG) of rod and cone responses before (WT-Baseline) and after ECVM application (WT-ECVM). For retinal responses measured by dark-adapted ERG, the a-wave (**FIGS. 4A and 4B**) and b-wave (**FIGS. 4C and 4D**) of WT-ECVM mice have normal amplitude and timing across intensity ranges as indicated. For light-adapted ERG, amplitude and implicit time of a-wave (**FIGS. 4E and 4F**) and b-wave (**FIGS. 4G and 4H**) in WT-ECVM eyes were not significantly different from baseline recordings across intensity ranges as shown.

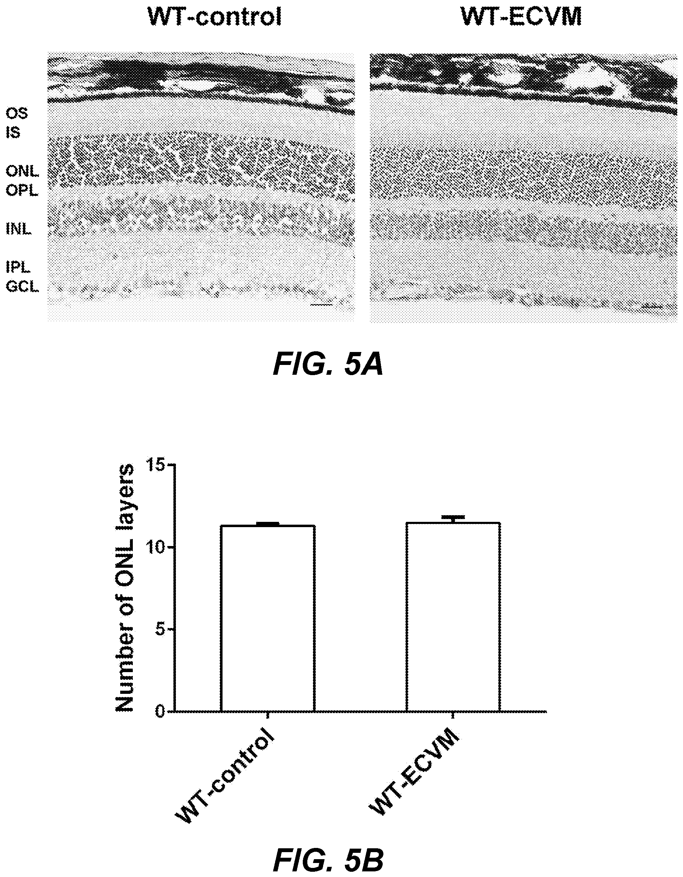

**FIGS. 5A** and **5B** show trans-ocular ECVM does not adversely affect retinal structure of wild type mice. Wild type mouse eyes (WT-ECVM, n=12) were treated with ECVM (monophasic, 10µA for 20 min) and retinal histology was examined 3 weeks later. Untreated wild type eyes (WT-control, n=4) served as controls. **FIG. 5A** shows retinal morphology by hematoxylin and eosin-stained sections cut through the optical nerve head (ONH) along the vertical meridian, indicating normal retinal structure of WT-ECVM mice. Scale bar, 25 µm. **FIG. 5B** shows the outer nuclear layer (ONL) thickness, evaluated by counting rows of ONL nuclei (ONL layers) across ONL width at 10 points along the retinal length of sections containing ONH. ONL thickness of WT-ECVM mice was indistinguishable from WT-control mice. OS - outer segments; IS - inner segments; ONL - outer nuclear layer; OPL - outer plexiform layer; INL - inner nuclear layer; IPL - inner plexiform layer; GCL - ganglion cell layer.

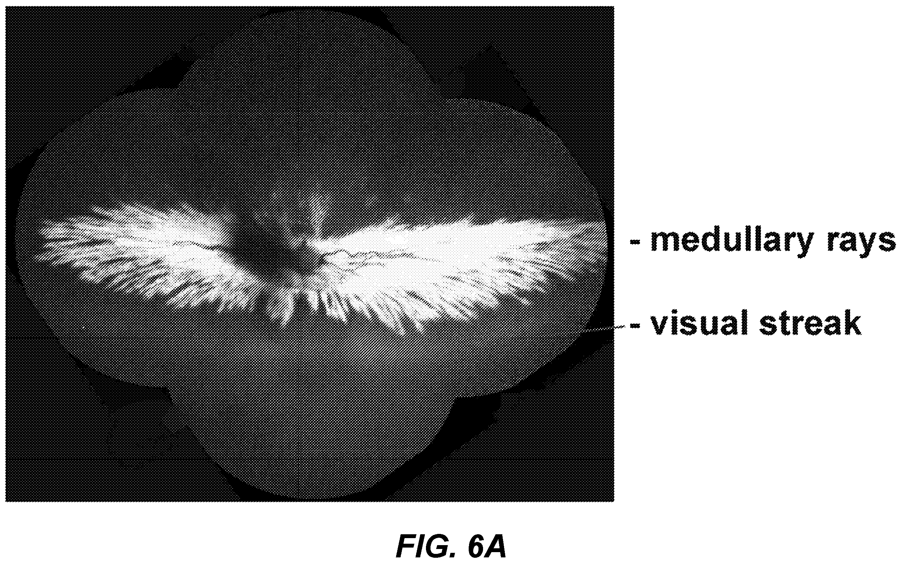

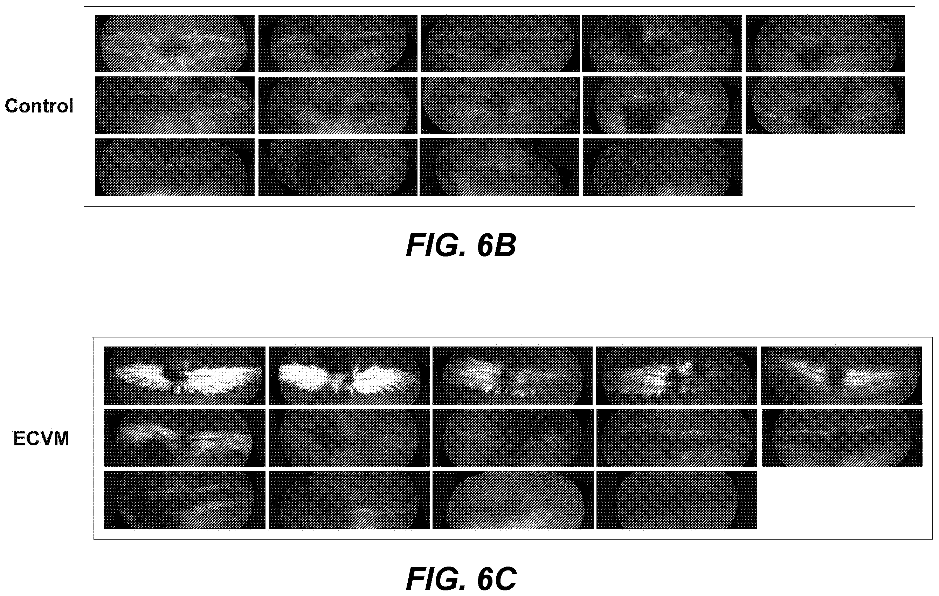

**FIGS. 6A-6C** show retinal AAV-GFP expression by fundus autofluorescence imaging in vivo following injection of 5X10¹¹ AAV-CMV-GFP vector genomes (vg) into the vitreous of the rabbit eye. **FIG. 6A** shows GFP expression monitored at 11 weeks post-injection in a specialized region of myelinated nerve fibers radiating from the optical nervehead, termed the "medullary rays." **FIG. 6B** shows GFP expression in eyes from control rabbits (no ECVM applied after injection of the vector genomes). **FIG. 6C** shows GFP expression in eyes from rabbits in which ECVM was applied after injection of the vector genomes.

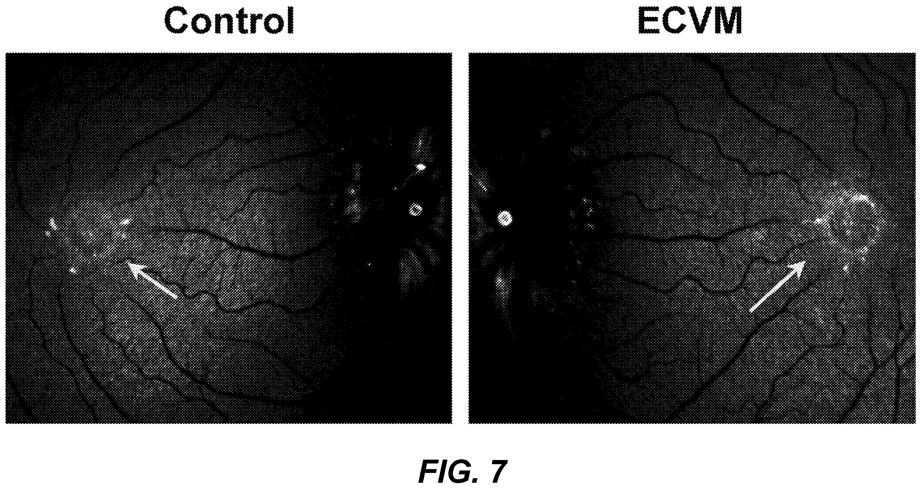

**FIG. 7** shows a comparison of GFP signal in the fovea (arrows) of rhesus monkey eyes that were injected intravitreal with an AAV8-CMV-EGFP (1e12 vg/dose) on both eyes. One eye (ECVM) received ECVM (850 µA/20min) following the intravitreal injection while the other eye received no ECVM (control).

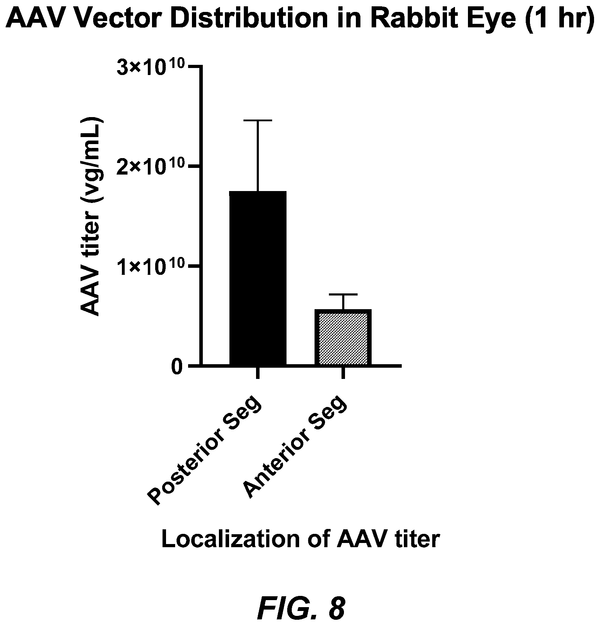

**FIG. 8** shows the distribution of vector genomes in rabbit eyes injected para-retinally with AAV8-CMV-EGFP vector. Each eye was administered 1.5 e11 vg of AAV8-CMV-EGFP placed next to the retina. One-hour post-administration, animals were sacrificed, and eyes collected. Each eye was frozen and hemi-sectioned into the posterior segment and anterior segment. AAV titer was determined by qPCR analyses. The error bars represent ± SEM (n=5).

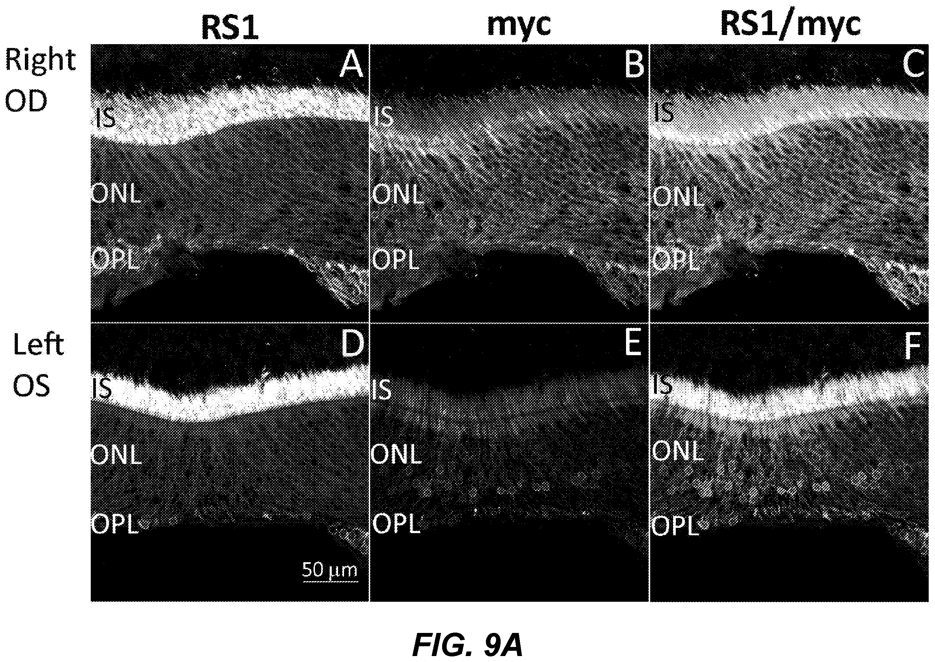

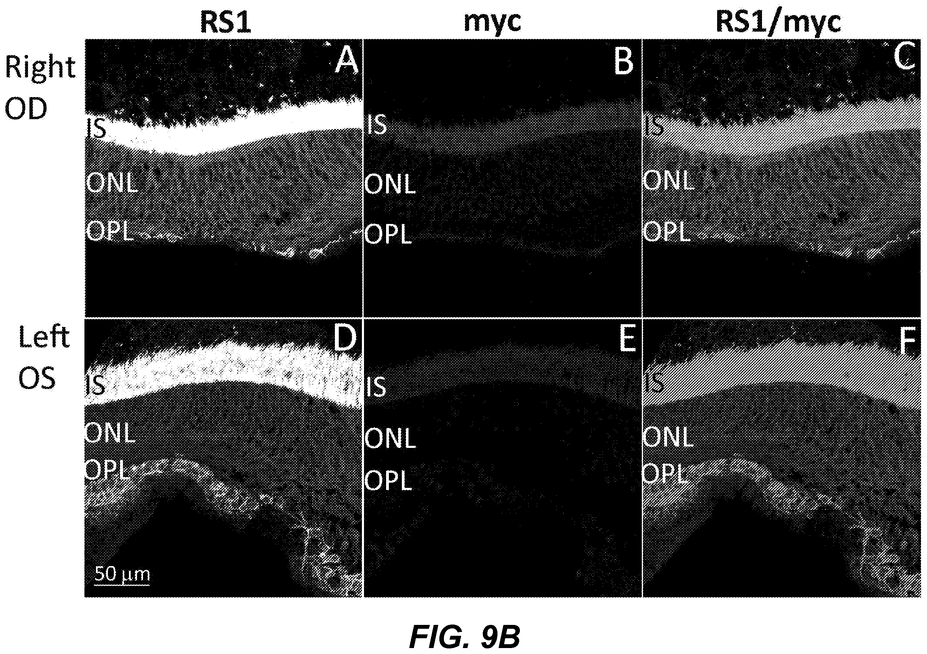

**FIGS. 9A** **and** **9B** show the effects of para-retinal and mid-vitreous delivery of gene therapy vectors into the eye of non-human primates. **FIG. 9A** shows the immunofluorescent assay of retinoschisin protein and RS1-myc tagged protein in Cynomolgus Monkey retinas following para-retinal delivery of scAAV8-HRSP/IRBP-hRS/myc. **FIG. 9B** shows the immunofluorescent assay of retinoschisin protein (RS1) and RS1-myc tagged protein in Cynomolgus Monkey retinas following mid-vitreous delivery of scAAV8-HRSP/IRBP-hRS/myc or vehicle.

### DETAILED DESCRIPTION

The present invention concerns a viral expression vector encoding a therapeutic protein for use in a method of treating an ophthalmic condition in a subject by intraocular delivery as defined in claim 1. The dependent claims define preferred features and embodiments of the present invention.

The scope of the present invention is defined by the appended claims.

Methods described hereinafter are not part of the present invention but are useful for understanding the present invention.

As used herein, the terms "individual," "subject," and "patient" are used interchangeably to refer to any human or other animal in need of treatment of a disease of the eye. Examples include, but are not limited to, humans and other primates, non-human primates such as chimpanzees and other apes and monkey species; farm animals such as cattle, sheep, pigs, seals, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. A preferred patient to treat is a human patient. The terms individual, subject, and patient by themselves, do not denote a particular age, sex, race, and the like. Thus, individuals of any age, whether male or female, are intended to be covered by the present disclosure and include, but are not limited to the elderly, adults, children, babies, infants, and toddlers. Likewise, the methods of the present disclosure can be applied to any race, including, for example, Caucasian (white), African-American (black), Native American, Native Hawaiian, Hispanic, Latino, Asian, and European.

As used herein, the term "efficacy" refers to the degree to which a desired effect is obtained. Specifically, the term refers to the degree to which a gene therapy vector is effective in treating an ocular disease or disorder following administration of the vector. The term "efficacy" as used in the context of the present disclosure also refers to relief or reduction of one or more symptoms or clinical events associated with an ocular disease or disorder.

The terms "administration of" or "administering an" expression vector should be understood to mean providing an expression vector to the subject in need of treatment in a form that can be introduced into that subject's body in a therapeutically useful form and therapeutically effective amount, including, but not limited to, injectable dosage forms, and nanoparticles, and the like.

The term "effective amount" as used herein refers to the amount necessary to elicit the desired biological response. As will be appreciated by those of ordinary skill in this art, the effective amount of a gene therapy vector may vary depending on such factors as the desired biological endpoint, the gene(s) to be delivered, the composition of the encapsulating matrix, the target tissue, etc.

The term "preventing", when used in relation to an ocular disease or disorder, is understood in the art, and includes administration of an expression vector which reduces the frequency of, or delays the onset of, symptoms of an ocular disease or disorder in a subject relative to a subject which does not receive the expression vector.

By "pharmaceutically acceptable" it is meant the carrier, diluent, or excipient must be compatible with the other ingredients of the formulation and not deleterious to the recipient thereof.

The term "synergistic" refers to two or more components working together so that the total effect is greater than the sum of the components.

The term "treating" refers to curing as well as ameliorating at least one symptom of any ocular condition or disorder.

The term "prophylactic treatment" includes administration to a subject of one or more therapeutic compositions (e.g., gene therapy vector) prior to clinical manifestation of the unwanted condition (e.g., disease or other unwanted state of the host animal) then the treatment is prophylactic, i.e., it protects the host against developing the unwanted ocular condition. If the therapeutic composition (e.g., gene therapy vector) is administered after manifestation of the ocular condition, the treatment is therapeutic (i.e., it is intended to diminish, ameliorate, or stabilize the existing unwanted condition or side effects thereof).

As used herein, an "expression vector" is a recombinant nucleic acid molecule comprising a nucleic acid sequence (e.g., open-reading frame (ORF)) that encodes a therapeutic molecule wherein the nucleic acid sequence is linked to a promoter that drives high level expression of the therapeutic molecule when the expression vector is administered to, for example, a subject or an organ, tissue or cell. An expression vector of the present disclosure is produced by human intervention and can be DNA, RNA or variants thereof. The expression vector may be a linear molecule (e.g., a linear nucleic acid molecule, a linear viral genome, etc.) or it may be a circular molecule, such as a plasmid. An expression vector may comprise one or more nucleic acid sequences from an adeno-associated virus (an AAV vector), a cytomegalovirus (CMV) (a CMV vector), a lentivirus, a retrovirus, an adenovirus, a herpes virus, a vaccinia virus (a vaccinia vector), a poliovirus, a Sindbis virus, or any other DNA or RNA virus. In embodiments, an expression vector may be a DNA plasmid. An expression vector may be a viral genome. An expression vector may be a DNA molecule, either linear or circular, comprising nucleic acid sequences from a plasmid and nucleic acid sequences from a viral genome to enable nucleic acid molecule delivery and high-level expression of the encoded therapeutic molecule. For example, the expression vector may be an AAV expression vector. As used herein, an AAV expression vector is a nucleic acid molecule comprising AAV sequences that allow for the replication, packaging and/or expression of the nucleic acid molecule. General methods for the construction of expression vectors are known in the art, and are also disclosed in, for example, Molecular Cloning: a Laboratory Manual, 3rd edition, Sambrook et al. 2001 Cold Spring Harbor Laboratory Press, and Current Protocols in Molecular Biology, Ausubel et al. eds., John Wiley & Sons, 1994.

The expression vector delivered within the administration methods of this disclosure can be administered to an eye of the subject either naked or in conjunction with a delivery reagent. Examples of delivery reagents for administration in conjunction with the methods of this disclosure include, but are not limited to, lipophilic reagents, lipofectin, lipofectamine, cellfectin, polycations (e.g., polylysine), micelles, PEGylated liposome or nanoparticles, oligonucleotide nanoparticles, cyclodextrin polymer (CDP)-based nanoparticles, biodegradable polymeric nanoparticles formulated with poly-(D,L-lactide-co-glycolide) (PLGA), Poly-lactic acid (PLA), or N-(2-hydroxypropyl)methacrylamide (HPMA), lipid nanoparticles (LNP), stable nucleic acid lipid particles (SNALP), vitamin A coupled lipid nanoparticles, and combinations thereof. These nanoparticles may be lipid nanoparticles that are conjugated to one or more moieties that target the tissues of the eye.

As noted above, expression vectors administered by the methods of the present disclosure comprise promoters that drive high-level expression of nucleic acid sequences encoding therapeutic molecules. As used herein, the phrase "drive expression" refers to the ability of a promoter to promote transcription from an open reading frame (ORF). According to the present disclosure, promoters used in expression vectors are specific for cells of the eye (i.e., eye-specific promoters). That is, the promoter only drives expression from the ORF when the expression vector is introduced into a cell of the eye. Such promoters are specific for cells, such as photoreceptor cells, bipolar cells, horizontal cells, amacrine cells, and ganglion cells. Examples of such promoters include, but are not limited to, a retinoschisin promoter, a rhodopsin promoter, a rhodopsin kinase promoter, a CRX promoter, and an interphotoreceptor retinoid binding protein (IRBP) promoter. Any promoter that allows eye-specific expression of an encoded protein can be used, so long as the promoter drives high-level expression of the ORF. Thus, the expression vector may comprise an eye-specific promoter.

As used herein, a therapeutic molecule is a molecule that when introduced within the eye, is capable or ameliorating or eliminating symptoms of a disease of the eye. Examples of therapeutic molecules include proteins and RNAs, including siRNAs. Such molecules may act by providing an activity that is missing, or significantly reduced, in a diseased eye. Such molecules may also act by modifying or reducing an activity that is over-expressed, or significantly elevated above normal levels, in a diseased eye. For example, a therapeutic molecule may be a protein possessing an activity (e.g., specific binding activity, enzymatic activity, transcriptional regulation activity, etc.) that is lacking in cells of the eye. Lack of such activity may result from failure of the cells to produce the protein, production of a mutated, inactive form of the protein, or misfolding of a protein resulting in an inactive form. In some cases, introducing a "good" (i.e., functional) copy of the protein may alleviate symptoms of the disease by directly replacing the missing activity. Alternatively, therapeutic molecules may act by increasing or decreasing the activity of other proteins in cells of the eye. For example, the therapeutic protein may bind to another protein and thereby either decrease or eliminate the activity of the second protein. Alternatively, binding of the therapeutic protein to another protein in cells of the eye may result in stabilization of such protein and/or an increase in the related activity. Finally, the therapeutic molecule may increase or decrease transcription of genes, or the translation of transcripts from genes in cells of the eye. For example, a therapeutic protein may bind to a transcriptional region of a gene and thereby increase or decrease transcription of that gene.

The present invention can be used to treat any monogenic retinal disease, including a disease of the eye in which the disease results from either inappropriate expression of a protein, or expression of a malfunctioning or dysfunctional form of a protein expressed in the eye. Inappropriate expression of a protein may refer to lack of expression, under-expression or over-expression of a protein. Expression of a malfunctioning form of a protein refers to expression of a protein having one or more mutation(s) that alters the activity of the protein. Alteration of activity may refer to complete inactivation of protein activity, reduction of protein activity or an increase in protein activity. Altered activity may result from, for example, direct inactivation of an active site or misfolding of the protein. Examples of eye diseases that may be treated using the administration methods of the present disclosure include, but are not limited to, Stargardt disease, X-linked retinoschisis, age-related macular degeneration (AMD), diabetic retinopathy, Leber congenital amaurosis (LCA), retinal detachment (due to disease, injury, or spontaneous), cysts, cystoid macular edema, retinitis pigmentosa, senile schisis, Fuchs corneal dystrophy and other corneal dystrophies, glaucoma, and cataract. Thus, the expression cassette may include polynucleotide sequences encoding proteins, therapeutic peptides, or protein fragments, such as ciliary neurotrophic factor (CNTF), brain-derived neurotrophic factor (BDNF), pigment epithelium-derived factor (PEDF), or pigment epithelium-derived factor (PEDF). For example, the inventors have tested a vector expressing lens epithelial derived growth factor (LEDGF) and demonstrated a protective effect in the RCS rat model of Retinitis pigmentosa (RP). For example, the eye disease treated using the methods of this disclosure may be X-linked retinoschisis (XLRS). X-linked retinoschisis is a neurodevelopmental retinal abnormally that causes impaired acuity and a propensity to retinal detachment. XLRS is characterized by structural abnormalities in normal lamination of the retinal neuronal and plexiform layers. Clinical examination shows microcysts within the macula, and schisis or internal dissection of the layers of the peripheral retina. X-linked juvenile retinoschisis is caused by mutations in the gene encoding retinoschisin, a 224-amino acid, secreted protein that is expressed only by the retina and pineal.

Any protein can be used as a therapeutic protein, provided the protein possesses an activity that is of therapeutic benefit in treating a disease of the eye. For example, if the disease to be treated is related to abnormal blood vessel growth (e.g., wet, age-related, macular degeneration (AMD), diabetic retinopathy, etc.) a useful therapeutic protein could be any protein having anti-angiogenic activity. As a further example, if the disease to be treated is due to neuropathy in the eye (e.g., glaucoma, retinitis pigmentosa, etc.) a useful therapeutic protein, may be any protein, or molecule, that provides a neuroprotective effect in the eye. Examples of such proteins include, but are not limited to, ciliary neurotrophic factor (CNTF), brain-derived neurotrophic factor (BDNF) and pigment epithelium-derived factor (PEDF).

Therapeutic proteins of the present disclosure may also be variants of wild-type proteins. As used herein, a variant refers to a protein, or nucleic acid molecule, the sequence of which is similar, but not identical to, a reference sequence, wherein the activity of the variant protein (or the protein encoded by the variant nucleic acid molecule) is not significantly altered. These variations in sequence can be naturally occurring variations, or they can be engineered through the use of genetic engineering technique known to those skilled in the art. Examples of such techniques may be found in Sambrook J, Fritsch E F, Maniatis T et al., in Molecular Cloning--A Laboratory Manual, 2nd Edition, Cold Spring Harbor Laboratory Press, 1989, pp. 9.31-9.57, or in Current Protocols in Molecular Biology, John Wiley & Sons, N.Y. (1989), 6.3.1-6.3.6.

With regard to variants, any type of alteration in the amino acid, or nucleic acid, sequence is permissible so long as the resulting variant protein retains the function of the wild-type protein. Examples of such variations include, but are not limited to, deletions, insertions, substitutions and combinations thereof. For example, with regard to proteins, it is well understood by those skilled in the art that one or more (e.g., 2, 3, 4, 5, 6, 7, 8, 9 or rh10), amino acids can often be removed from the amino and/or carboxy terminal ends of a protein without significantly affecting the activity of that protein. Similarly, one or more (e.g., 2, 3, 4, 5, 6, 7, 8, 9 or rh10) amino acids can often be inserted into a protein without significantly affecting the activity of the protein.

One example of a useful therapeutic protein is retinoschisin protein, which is a 224-amino acid, discoidin domain-containing, retina-specific, secretory protein. Loss of retinoschisin protein function has been implicated in X-linked retinoschinosis. As used herein, a retinoschisin protein refers to a full-length retinoschisin protein, or any portion thereof, that has at least one activity of a wild-type retinoschisin protein. Thus, the therapeutic protein may comprise at least a portion of a retinoschisin protein. Such a portion may comprise at least 50 amino acids, at least 75 amino acids, at least 125 amino acids, at least 150 amino acids, at least 175 amino acids or at least 200 amino acids, so long as the resulting therapeutic protein possesses at least one function of a full length retinoschisin protein. In a related example, the therapeutic protein is a retinoschisin protein having at least 90%, at least 95%, at least 97%, at least 98% or at least 99% sequence identity to a full-length retinoschisin protein, or any portion thereof, that has at least one activity of a wild-type retinoschisin protein. In a specific example, the therapeutic protein is a human retinoschisin protein having at least 90%, at least 95%, at least 97%, at least 98% or at least 99% sequence identity to a full-length human retinoschisin protein, or any portion thereof, that has at least one activity of a wild-type retinoschisin protein. Known functions of the retinoschisin protein include binding to anionic phospholipids, binding to the sterile alpha and TIR motif-containing protein (SARM1), binding to alpha-B crystalline protein and binding to beta2 laminin.

The expression vectors delivered within the administration methods of this disclosure may comprise nucleic acid sequences that allow replication of the vector by viral systems and packaging of the expression vector into a viral vector. One example of such sequences is the inverted terminal repeat (ITR) sequences found in adeno-associated viruses. Examples of viral replication systems are known in the art and include, for example, the use of helper viruses (e.g., adenoviruses) as well as recombinant cells expressing proteins that recognize AAV ITR sequences and direct replication of nucleic acid molecules comprising ITR sequences (e.g., cells expressing AAV Rep proteins). Similarly, packaging systems that can package the expression vector into viral vectors are known to those in the art (e.g., recombinant cells expressing AAV capsid proteins). Thus, in embodiments, the expression vector comprises at least one AAV ITR sequence. In embodiments, the expression vector comprises a pair of AAV ITR sequences. AAV ITR sequences useful for constructing expression vectors of the present invention can be from any AAV so long as they are capable of allowing replication of the expression vector by an AAV replication system, and packaging of the expression vector into a viral vector. In embodiments, the expression vector comprises at least one ITR sequence from a virus selected from the group consisting of AAV1, AAV2, AAV4, AAV5, AAV7, AAV8, AAV9, and AAVrh10. In embodiments, the expression vector comprises at least one ITR sequence from AAV8.

As has been discussed, packaging of the expression vector into a viral vector may increase the efficiency of delivery of the expression vector into cells of the eye. As used herein, a viral vector refers to a particle that comprises capsid proteins from one or more viruses, and which can encapsulate, or contain, the expression vector within the particle. Viral vectors may increase the efficiency of delivery by binding to receptors on the cell surface and becoming internalized (e.g., by fusion with the cell membrane or by endocytosis) thereby delivering the expression vector into the interior of cells of the eye. The capsid proteins of any virus can be used to construct viral vectors, so long as the resulting viral vector is capable of delivering the expression vector into cells of the eye. Preferred capsid proteins to be used in constructing viral vectors may be obtained from a virus selected from the group consisting of an adeno-associated virus (an AAV virus), a cytomegalovirus (CMV), a retrovirus, an adenovirus, a herpes virus, a vaccinia virus, a poliovirus, and a Sindbis virus.

Currently there are several known AAVs, examples of which include AAV1, AAV2, AAV3, AAV4, AAV5, AAV7, AAV8, AAV9, and AAVrh10. The capsid protein from any AAV can be used so long as the resulting particle is able to encapsulate an expression vector of the present invention and deliver it into cells of the eye. In a preferred embodiment, the capsid proteins are from AAV8. Thus, one embodiment of the present invention is a method of administering a viral vector comprising capsid proteins from AAV8 (an AAV8 vector), wherein the viral vector comprises an expression vector. Particularly preferred expression vectors, and viral vectors encompassing such expression vectors, are described in International Application No. PCT/US2014/016389 (WIPO Publication No. WO/2014/127196, 21 August 2014).

In these methods, the expression vector, either alone or in an encapsulated form, or incorporated into a nanoparticle, is injected into the eye. This may include intramuscular, intradermal, subcutaneous, subconjunctival and sub-Tenon's, intravitreal, subretinal, intravenous and intracameral injections. Such injections can deliver the expression vector, or a viral vector containing the expression vector, to the intraocular fluid or to a location within the retina. In embodiments, the injection delivers the expression vector, or a viral vector containing the expression vector, to the intraocular fluid. In embodiments, the injection delivers the expression vector, or a viral vector containing the expression vector, into the retina. In embodiments, the expression vector is administered by intravitreal injection. In another embodiment, the expression vector is administered by subretinal injection. In another embodiment, the expression vector is administered by sub-Tenon's injection. Methods of performing intraocular injections are known to those skilled in the art. In all these methods, the expression vector is preferably contained within and administered via a polypropylene syringe. When administered by these means, the single injection dosage may include between 1e⁸ vg/eye and 3e¹³ vg/eye (i.e., 1 x 10⁸ vector genomes (vg) per eye to 3 x 10¹³ vector genomes per eye). When administered by these means, the single injection dosage may be between 3e⁸ vg/eye and 1e¹³ vg/eye, or between 1e⁹ vg/eye and 1e¹³ vg/eye, or between 3e⁹ vg/eye and 1e¹³ vg/eye, or between 1e¹⁰ vg/eye and 1e¹³ vg/eye, or between 3e¹⁰ vg/eye and 1e¹³ vg/eye, or between 1e¹¹ vg/eye and 1e¹³ vg/eye, or between 3e¹¹ vg/eye and 1e¹³ vg/eye, or between 1e¹² vg/eye and 1e¹³ vg/eye, or between 3e¹² vg/eye and 1e¹³ vg/eye.

In embodiments of the administration methods of this disclosure, the electric current may be a low level electric current, for example, between about 1 and about 200 micro-amperes, between about 10 and about 190 micro-amperes, between about 20 and about 180 micro-amperes, between about 30 and about 170 micro-amperes, between about 40 and about 160 micro-amperes, between about 50 and about 150 micro-amperes, between about 60 and about 140 micro-amperes, between about 70 and about 130 micro-amperes, between about 80 and about 120 micro-amperes, between about 90 and about 100 micro-amperes, between about 100 and about 150 micro-amperes, between about 150 and about 200 micro-amperes, between about 200 and about 250 micro-amperes, between about 250 and about 300 micro-amperes, between about 300 and about 350 micro-amperes, between about 350 and about 400 micro-amperes, between about 400 and about 450 micro-amperes, between about 450 and about 500 micro-amperes, between about 500 and about 550 micro-amperes, between about 550 and about 600 micro-amperes, between about 600 and about 650 micro-amperes, between about 650 and about 700 micro-amperes, between about 700 and about 750 micro-amperes, between about 750 and about 800 micro-amperes, between about 800 and about 850 micro-amperes, between about 850 and about 900 micro-amperes, between about 900 and about 950 micro-amperes, between about 950 and about 1000 micro-amperes (1 milli-amp [mA]), between about 1.0 and about 1.1 mA, between about 1.1 and about 1.2 mA, between about 1.2 and about 1.3 mA, between about 1.3 and about 1.4 mA, between about 1.4 and about 1.5 mA, between about 1.5 and about 1.6 mA, between about 1.6 and about 1.7 mA, between about 1.7 and about 1.8 mA, between about 1.8 and about 1.9 mA, between about 1.9 and about 2.0 mA, between about 2.0 and about 2.1 mA, between about 2.1 and about 2.2 mA, between about 2.2 and about 2.3 mA, between about 2.3 and about 2.4 mA, between about 2.4 and about 2.5 mA, between about 2.5 and about 2.6 mA, between about 2.6 and about 2.7 mA, between about 2.7 and about 2.8 mA, between about 2.8 and about 2.9 mA, between about 2.9 and about 3.0 mA, between about 3.0 and about 3.1 mA, between about 3.1 and about 3.2 mA, between about 3.2 and about 3.3 mA, between about 3.3 and about 3.4 mA, between about 3.4 and about 3.5 mA, between about 3.5 and about 3.6 mA, between about 3.6 and about 3.7 mA, between about 3.7 and about 3.8 mA, between about 3.8 and about 3.9 mA, between about 3.9 and about 4.0 mA, between about 4.0 and about 4.1 mA, between about 4.1 and about 4.2 mA, between about 4.2 and about 4.3 mA, between about 4.3 and about 4.4 mA, between about 4.4 and about 4.5 mA, between about 4.5 and about 5.0 mA, between about 5.0 and about 5.5 mA, between about 5.5 and about 6.0 mA, between about 6.0 and about 6.5 mA, between about 6.5 and about 7.0 mA, between about 7.5 and about 8.0 mA, between about 8.0 and about 8.5 mA, between about 8.5 and about 9.0 mA, between about 9.0 and about 9.5 mA, between about 9.5 and about 10.0 mA, between about 10.0 and about 10.5 mA, between about 10.5 and about 11.0 mA, between about 11.0 and about 11.5 mA, between about 11.5 and about 12.0 mA, between about 12.0 and about 12.5 mA, between about 12.5 and about 13.0 mA, between about 13.0 and about 13.5 mA, between about 13.5 and about 14.0 mA, between about 14.0 and about 14.5 mA, between about 14.5 and about 15.0 mA, or the like.

In embodiments of the administration methods of this disclosure, the electric current may be a low level electric current of between for example about 1 and about 400 micro-amperes, between about 20 and about 380 micro-amperes, between about 40 and about 360 micro-amperes, between about 60 and about 340 micro-amperes, between about 80 and about 320 micro-amperes, between about 100 and about 3000 micro-amperes, between about 120 and about 280 micro-amperes, between about 140 and about 260 micro-amperes, between about 160 and about 240 micro-amperes, between about 180 and about 220 micro-amperes, or the like.

In embodiments of the administration methods of this disclosure, the electric current may be a low level electric current of between, for example, about 1 micro-ampere and about 1 milli-ampere, between about 50 and about 800 micro-amperes, between about 200 and about 600 micro-amperes, between about 400 and about 500 micro-amperes, or the like.

In embodiments of the administration methods of this disclosure, the electric current may be a low level electric current of about 10 micro-amperes, about 20 micro-amperes, about 30 micro-amperes, about 40 micro-amperes, about 50 micro-amperes, about 60 micro-amperes, about 70 micro-amperes, about 80 micro-amperes, about 90 micro-amperes, about 100 micro-amperes, about 110 micro-amperes, about 120 micro-amperes, about 130 micro-amperes, about 140 micro-amperes, about 150 micro-amperes, about 160 micro-amperes, about 170 micro-amperes, about 180 micro-amperes, about 190 micro-amperes, about 200 micro-amperes, about 210 micro-amperes, about 220 micro-amperes, about 240 micro-amperes, about 260 micro-amperes, about 280 micro-amperes, about 300 micro-amperes, about 320 micro-amperes, about 340 micro-amperes, about 360 micro-amperes, about 380 micro-amperes, about 400 micro-amperes, about 450 micro-amperes, about 500 micro-amperes, about 550 micro-amperes, about 600 micro-amperes, about 650 micro-amperes, about 700 micro-amperes, about 750 micro-amperes, about 800 micro-amperes, about 850 micro-amperes, about 900 micro-amperes, about 950 micro-amperes, about 1 milli-ampere, or the like.

In embodiments of the administration methods of this disclosure, the electric current may be a low level electric current of not more than about 10 micro-amperes, or not more than about 20 micro-amperes, not more than about 30 micro-amperes, not more than about 40 micro-amperes, not more than about 50 micro-amperes, not more than about 60 micro-amperes, not more than about 70 micro-amperes, not more than about 80 micro-amperes, not more than about 90 micro-amperes, not more than about 100 micro-amperes, not more than about 110 micro-amperes, not more than about 120 micro-amperes, not more than about 130 micro-amperes, not more than about 140 micro-amperes, not more than about 150 micro-amperes, not more than about 160 micro-amperes, not more than about 170 micro-amperes, not more than about 180 micro-amperes, not more than about 190 micro-amperes, not more than about 200 micro-amperes, not more than about 210 micro-amperes, not more than about 220 micro-amperes, not more than about 230 micro-amperes, not more than about 240 micro-amperes, not more than about 250 micro-amperes, not more than about 260 micro-amperes, not more than about 270 micro-amperes, not more than about 280 micro-amperes, not more than about 290 micro-amperes, not more than about 300 micro-amperes, not more than about 310 micro-amperes, not more than about 320 micro-amperes, not more than about 340 micro-amperes, not more than about 360 micro-amperes, not more than about 380 micro-amperes, not more than about 400 micro-amperes, not more than about 420 micro-amperes, not more than about 440 micro-amperes, not more than about 460 micro-amperes, not more than about 480 micro-amperes, not more than about 500 micro-amperes, not more than about 520 micro-amperes, not more than about 540 micro-amperes, not more than about 560 micro-amperes, not more than about 580 micro-amperes, not more than about 600 micro-amperes, not more than about 620 micro-amperes, not more than about 640 micro-amperes, not more than about 660 micro-amperes, not more than about 680 micro-amperes, not more than about 700 micro-amperes, not more than about 720 micro-amperes, not more than about 740 micro-amperes, not more than about 760 micro-amperes, not more than about 780 micro-amperes, not more than about 800 micro-amperes, not more than about 820 micro-amperes, not more than about 840 micro-amperes, not more than about 860 micro-amperes, not more than about 880 micro-amperes, not more than about 900 micro-amperes, not more than about 920 micro-amperes, not more than about 940 micro-amperes, not more than about 960 micro-amperes, not more than about 980 micro-amperes, or the like.

In embodiments of the administration methods of this disclosure, the electric current may be a low level electric current of not less than 10 micro-amperes, not less than 20 micro-amperes, not less than 30 micro-amperes, not less than 40 micro-amperes, not less than 50 micro-amperes, not less than 60 micro-amperes, not less than 70 micro-amperes, not less than 80 micro-amperes, not less than 90 micro-amperes, not less than 100 micro-amperes, not less than 110 micro-amperes, not less than 120 micro-amperes, not less than 130 micro-amperes, not less than 140 micro-amperes, not less than 150 micro-amperes, not less than 160 micro-amperes, not less than 170 micro-amperes, not less than 180 micro-amperes, not less than 190 micro-amperes, not less than 200 micro-amperes, not less than 210 micro-amperes, not less than 220 micro-amperes, not less than 230 micro-amperes, not less than 240 micro-amperes, not less than 250 micro-amperes, not less than 260 micro-amperes, not less than 270 micro-amperes, not less than 280 micro-amperes, not less than 290 micro-amperes, not less than 300 micro-amperes, not less than 310 micro-amperes, not less than 320 micro-amperes, not less than 330 micro-amperes, not less than 340 micro-amperes, not less than 350 micro-amperes, not less than 360 micro-amperes, not less than 370 micro-amperes, not less than 380 micro-amperes, not less than 390 micro-amperes, not less than 400 micro-amperes, not less than about 420 micro-amperes, not less than about 440 micro-amperes, not less than about 460 micro-amperes, not less than about 480 micro-amperes, not less than about 500 micro-amperes, not less than about 520 micro-amperes, not less than about 540 micro-amperes, not less than about 560 micro-amperes, not less than about 580 micro-amperes, not less than about 600 micro-amperes, not less than about 620 micro-amperes, not less than about 640 micro-amperes, not less than about 660 micro-amperes, not less than about 680 micro-amperes, not less than about 700 micro-amperes, not less than about 720 micro-amperes, not less than about 740 micro-amperes, not less than about 760 micro-amperes, not less than about 780 micro-amperes, not less than about 800 micro-amperes, not less than about 820 micro-amperes, not less than about 840 micro-amperes, not less than about 860 micro-amperes, not less than about 880 micro-amperes, not less than about 900 micro-amperes, not less than about 920 micro-amperes, not less than about 940 micro-amperes, not less than about 960 micro-amperes, not less than about 980 micro-amperes, or the like.

In embodiments of the administration methods of this disclosure, the electric current may be applied for a time period of between 1 millisecond (ms) and 1000 ms, between 5 ms and 995 ms, between 10 ms and 900 ms, between 20 ms and 800 ms, between 25 ms and 750 ms, between 30 ms and 700 ms, between 35 ms and 650 ms, between 40 ms and 600 ms, between 45 ms and 500 ms, between 50 ms and 500 ms, and the like.

In embodiments of the administration methods of this disclosure, the electric current may be applied for a time period of between 1 second (s) and 60 s, between 2 s and 58 s, between 4 s and 56 s, between 6 s and 54 s, between 8 s and 52 s, between 10 s and 50 s, between 12 s and 48 s, between 14 s and 46 s, between 16 s and 44 s, between 18 s and 42 s, between 20 s and 40 s, between 22 s and 38 s, between 24 s and 36 s, between 26 s and 34 s, between 28 s and 32 s, and the like.

In embodiments of the administration methods of this disclosure, the electric current may be applied for a time period of between 1 minute (min) and 60 min, between 2 min and 58 min, between 4 min and 56 min, between 6 min and 54 min, between 8 min and 52 min, between 10 min and 50 min, between 12 min and 48 min, between 14 min and 46 min, between 16 min and 44 min, between 18 min and 42 min, between 20 min and 40 min, between 22 min and 38 min, between 24 min and 36 min, between 26 min and 34 min, between 28 min and 32 min, and the like.

In embodiments of the administration methods of this disclosure, the electric current may be applied for a time period of between 1 hour (hr) and 24 hr, between 2 hr and 22 hr, between 4 hr and 20 hr, between 6 hr and 18 hr, between 8 hr and 16 hr, between 10 hr and 14 hr, between 12 hr and 13 hr, and the like.

In embodiments of the administration methods of this disclosure, the electric current may be applied to the eye currently with the injection of the expression vector. As a practical matter, this administration timing may encompass the application of the electric current in a time period between 1 minute before to within 1 minute after injection of the expression vector.

In embodiments of the administration methods of this disclosure, the electric current may be applied to the eye before the injection of the expression vector. In these methods, the electric current may be applied to the eye up to 48 hours before injection of the expression vector. In these methods, the electric current may be applied to the eye in a time period between 24 hours and 48 hours before injection of the expression vector, or between 12 hours and 24 hours before injection of the expression vector, or between 6 hours and 12 hours before injection of the expression vector, or between 1 hour and 6 hours before injection of the expression vector, or between 1 minute and 60 minutes before injection of the expression vector.

In other embodiments of the administration methods of this disclosure, the electric current may be applied to the eye after the injection of the expression vector. In these methods, the electric current may be applied to the eye up to 48 hours after injection of the expression vector. In these methods, the electric current may be applied to the eye in a time period between 24 hours and 48 hours after injection of the expression vector, or between 12 hours and 24 hours after injection of the expression vector, or between 6 hours and 12 hours after injection of the expression vector, or between 1 hour and 6 hours after injection of the expression vector, or between 1 minute and 60 minutes after injection of the expression vector.

Electrodes of various types can be used to produce the applied electric currents, including, for example: (1) inert electrodes and (2) active electrodes. Active electrodes require aqueous media containing electrolytes. The electrical polarity of the drug delivery electrode may be chosen depending upon the chemical nature of the expression vector or nanoparticle, specifically its pKa(s)/isoelectric point and the initial dosing solution pH. The electrochemical repulsion between the ions generated via electrolysis and the expression vector's charge may drive the expression vector into tissues. Thus, administration in conjunction with the application of an electric current offers a significant advantage over topical drug application, in that it increases drug absorption. The rate of drug delivery may be adjusted by varying the applied current or time, as determined by one of skill in the art.

Inert electrodes useful in the administration methods of this disclosure include electrodes comprising any electrically-conductive material, including, but not limited to, copper metal, and/or silver metal, and/or gold metal electrodes, and/or platinum, and or mercury, and or carbon electrodes.

The gene therapy vectors administered within the methods of this disclosure may be administered to subjects (e.g., humans and animals, including companion animals, such as dogs, cats and horses) in need of such treatment in dosages that will provide optimal pharmaceutical efficacy. It will be appreciated that the dose required for use in any particular application will vary from patient to patient, not only with the particular expression vector or composition selected, but also with the route of administration, the nature of the condition being treated, the age and condition of the patient, concurrent medication or special diets then being followed by the patient, and other factors which those skilled in the art will recognize, with the appropriate dosage ultimately being at the discretion of the attendant medical professional.

Several interdependent factors influence the overall efficacy and safety of a gene therapy vector preparation in the treatment of ocular disease or disorders. These include the ability of a gene therapy vector to penetrate through the ILM, the cornea, sclera or, blood-ocular barrier, the relative expression efficiency, duration of action, and distribution in the target tissue, the dose and frequency of administration, the adverse event profile, and the site of administration. Preferably, the gene therapy vector is initially injected in a location close to the tissue being targeted: for retina, intra-vitreal injections are preferred, as described above; and this may also be a good location to reach the back of the lens and the ciliary body. For the corneal endothelium (a site of very high disease frequency, such as in Fuchs endothelial corneal dystrophy), the lens, and also for the trabecular meshwork (involved in glaucoma with buildup of intraocular pressure), injecting the vector into the anterior chamber of the eye is a preferred location.

A pertinent parameter affecting the delivery efficacy of a gene therapy vector composition is the pH of the administered composition. Various pH ranges are obtained by pH adjustment with acid or base using various buffering systems including, for example, phosphate buffers; the conductivity of the administered composition. Various conductivity ranges are obtained by altering the salt (e.g., NaCl, KCI, etc.) concentration; osmolarity of the administered composition. Various osmolarity ranges are obtained by addition of, for example, mannitol; ionic strength. Various ionic strengths are obtained by the addition of various ionic compounds (e.g., NaCl, KCI, CaCl₂, MgCl₂, etc.); viscosity of the administered composition. Various viscosities are obtained by the addition of, for example, various polyethylene glycol species (PEG's).

Other parameters that are considered in optimizing delivery of an expression vector by the methods of this disclosure include, for example, use of inert versus active electrodes, choice of buffer system, choice of excipients (possibly required for adjusting osmolarity), expression vector charge (e.g., pKa and pl), expression vector solubility, expression vector concentration, expression vector stability, choice of drug stabilizer, cosolvents, and emulsions.

Application of the electric current within the methods of this disclosure may generally include placing a simple electrode on the cornea or in the eye (for example, a single wire as the negative electrode) and placing another simple electrode anywhere on the body of the subject or elsewhere on the eye. The goal of choosing the placement of the electrodes is to get current to flow through the eye thereby allowing entry of the expression vector into the desired tissue in the eye, for example, to the retina in the posterior portion of the eye, or to the cornea, lens, iris, trabecular meshwork, or ciliary body in the anterior portion of the eye. Thus, it is also possible to place two electrodes on the eye, with one or both or neither electrode contacting the cornea, such that the applied current passes across the entire eye. The electrode placement may also include inserting an electrode into the eye, which would apply current closer to the retina and may therefore have greater affect in enhancing delivery of the gene expression. For example, this is done for intra-dermal electrodes in which only the tip is exposed metal and the shaft is coated with Teflon (or other) insulation, and such electrode materials and set up could easily be used in the administration methods of this disclosure. In such cases, a needle electrode would penetrate the eye, preferably positioned in the vitreous, while a second electrode would be outside the eye, including "grounding" the body. In these methods, a current could be applied to an ocular surgical instrument placed inside the vitreous thereby acting as the electrode inserted into the eye. The electrode on the body is essentially a "common" or "ground" and the electrode placed on the eye is a point from which the current extends out more or less radially.

The current may be applied before or after application of the expression vector or nanoparticle into the vitreous cavity of the eye. For example, the current may be applied as much as 48 hours before injection of the expression vector or nanoparticle into the eye. Thus, the current may be applied to the eye for a period between 1 hour and 48 hours before injection, or between 30 minutes and 24 hours before injection, or between 30 seconds and 12 hours before injection, or between 30 milliseconds and 6 hours before injection of the expression vector or nanoparticle into the eye. In another example, the current may be applied as much as 48 hours after injection of the expression vector or nanoparticle into the eye. Thus, the current may be applied to the eye for a period between 1 hour and 48 hours after injection, or between 30 minutes and 24 hours after injection, or between 30 seconds and 12 hours after injection, or between 30 milliseconds and 6 hours after injection of the expression vector or nanoparticle into the eye. During application, the electric current is preferably monitored. Of course, one of skill in the art will appreciate the control and consistency of using a constant-current power supply, which can precisely apply and/or control and/or monitor an electric current in the micoamp to milliamp range.

The parameters related to the administration of the expression vector and the application of the electric current, described above, control the direction of electric current between the two electrodes. For example, in an exemplary embodiment, a negative electrode may be placed on the cornea, and a positive electrode is placed subcutaneously. In a related embodiment, the charge on the electrodes may be reversed (i.e., a positive electrode placed on the cornea, and a negative electrode placed subcutaneously). This may allow the expression vector/particle to enter tissues of the crystalline lens, the iris, the ciliary body, and/or the cornea, thereby enhancing transfection of those tissues and ocular structures in the anterior portion of the eye for therapy.

The present disclosure also relates to kits for practicing the methods of this disclosure. Kits of this disclosure may comprise expression vectors and viral vectors effective in the treatment of diseases or disorders of the eye. Such kits may also comprise reagents and tools necessary for practicing the disclosed methods, such as diluents, syringes, needles, apparatus (or components of an apparatus) for delivering the compound into the eye of a subject, and instructions for administering such reagents. These kits may also include one or more gene therapy vectors suitable for delivery of a gene therapy construct encoding a therapeutic protein to the eye of a subject.

The scope of the present invention invention is defined by the appended claims.

### EXAMPLES

The following methods were used to conduct experiments described in Examples 1-4, below:
**Animals:** Adult wild-type C57BL/6J mice 8 -12 weeks old (Jackson Laboratory, Bar Harbor, ME) were used in the mouse experiments. Mice were housed in nominally 60 lux dim white fluorescent lighting on a 12h/12h light/dark cycle. Experimental protocols were approved by the NIH Animal Care and Use Committee and adhered to the ARVO Statement for the Use of Animals in Ophthalmic and Vision Research. For all procedures, anesthesia was performed with intraperitoneal ketamine (92.6 mg/kg)/xylazine (5.6 mg/kg) solution; and pupils were dilated with topical ocular 0.5% tropicamide (Alcon Laboratories) and 0.5% phenylephrine hydrochloride (Bausch & Lomb) after topical 0.5% tetracaine was applied to the eye.
Retinal AAV-GFP expression was evaluated by fundus imaging in vivo using a Spectralis fundus camera (Heidelberg Engineering, Heidelberg, Germany). Eyes were harvested at the termination of the study, and retinal immunohistochemistry was performed to assess the distribution of retinal cells transduced by the AAV8-EGFP in current-treated and in no-current eyes.
**Adeno-associated virus serotype 8 (AAV8) vector:** The AAV8-CMV-EGFP vector has a cytomegalovirus (CMV) promoter, a chimeric CMV/human β-globin intron, the enhanced green fluorescent protein (EGFP) gene and a human β-globin polyadenylation (PolyA) site. Recombinant AAV was produced by the triple transfection method and purified by polyethylene glycol precipitation followed by cesium chloride density-gradient fractionation, as previously described (Park T. K., et al., (2009). Intravitreal delivery of AAV8 retinoschisin results in cell type-specific gene expression and retinal rescue in the Rs1-KO mouse. Gene Ther. 16: 916-926). The purified AAV vectors were formulated in 10 mM Tris-HCl, 180 mM NaCl pH 7.4 and 0.001% Pluronic F-68 pH 7.4 and stored at -80°C. Quantification of vectors was done by real time PCR using linearized plasmid standards.
**Intravitreal injection and ocular electric current (ECVM) application:** AAV8-CMV-EGFP vector dilutions 5 X 10⁸ or of 1 X 10⁹ vg/eye were administered by intravitreal injection to adult WT mice. Mice were first anesthetized, and pupils dilated. Animals were positioned under a dissecting microscope with the injected eye facing upward. AAV8-EGFP 1 µl suspension was injected into the vitreous through the nasal sclera approximately 1 mm posterior to the limbus using a 35-gauge beveled-tip needle attached to a 10-µL Nanofil syringe (World Precision Instruments, Inc., Sarasota, FL, USA). Triple antibiotic ophthalmic ointment of neomycin, polymyxin B, and bacitracin was applied to the eye. Injected eyes then received electric current (ECVM) application immediately (approx. 5 minutes) after injection. Animals were kept on a warming pad at 32-33°C. Injected eyes without electric current application served as controls. Due to the very small size of the mouse eye and vitreous cavity, retinal injury sometimes occurred by touching the retina with the needle tip opposite the entry site. When we observed evident retinal damage on OCT, some of these eyes showed widespread GFP expression. We excluded those eyes from our analysis. This occurred in 6 eyes of 65 eyes injected.
**Trans-ocular ECVM application.** Mice were anesthetized, and a gold wire ring electrode was placed on the center of cornea with a thin layer of GONAK Hypromellose (Akorn, Inc.) or phosphate buffered saline (PBS) to maintain corneal moisture. Care was taken to use minimal fluid on the cornea so as not to electrically short circuit the cornea to the lid. A subdermal needle electrode was inserted subcutaneously on the forehead above the same eye lid. The two electrodes were separated by approximately 1 cm. Previous studies of trans-ocular electric current for neuroprotection used biphasic 1.5 to 300 micro-amperes (µA) for 30 - 60 minutes which was safe for the rodent eye with retinal degeneration (Pardue M. T., et al., (2014). Neuroprotective effects of low level electrical stimulation therapy on retinal degeneration. Adv Exp Med Biol. 801:845-851). We initially tested 10 µA and 50 µA continuous direct current (DC) for 20 min with the negative electrode on the cornea using a constant current stimulus isolator (A365, World Precision Instruments). These currents were achieved with less than 3 or 4 volts applied. 50 µA current application caused cornea scaring adjacent to the electrode ring and occasional cataract, while 10 µA current caused no complications. To evaluate retinal safety, baseline electroretinogram (ERG) recordings were made in 7 mice, and 10 µA DC current was applied to the cornea for 20 min one week later. ERG function was tested again 3 weeks after electric current application. Compared to baseline, retinal ERG function of rod and cone responses showed no change to amplitude or timing after 10 µA DC current, and the intensity-response curves of a- and b-wave responses were unchanged. Eyes were removed 3 days later for retinal histology and both morphology and ONL thickness were unaffected compared with untreated C57BL/6J mice which served as controls. The results of retinal ERG, morphology and ONL thickness are shown in (**FIGS. 4A-4H** and **FIGS. 5A, 5B**). We selected the 10 µA current for further testing in mice.
**GFP expression by *in vivo* fundus imaging:** Four to six weeks after intravitreal AAV8- EGFP application, GFP expression was evaluated by fundus imaging *in vivo* (Spectralis fundus camera, Heidelberg Engineering). Mice were anesthetized, and pupils dilated. Blue fluorescence fundus images were captured with ultra-widefield (102 degrees) lens. The GFP-positive area of the central retina was measured using an intensity threshold method with ImageJ software (imagej.nih.gov/ij). In brief, the analysis used photographs with the optic disc at the center, and an intensity threshold was applied to select GFP-positive areas from background signals. The same threshold setting was used for all analyses. The area percentage of GFP expression in the graph was calculated by dividing the area of GFP signal in the central retina by the total area of the central retina.
**GFP intensity and distribution determined on retinal immunohistochemistry:** Retinal immunohistochemistry was performed as previously described (Song H., et al. (2016). NADPH Oxidase Contributes to Photoreceptor Degeneration in Constitutively Active RAC1 Mice. Invest Ophthalmol Vis Sci. 57: 2864-75). Briefly, eyes were fixed, cryoprotected, embedded, snap-frozen in Tissue-Tek O.C.T. compound (Sakura Finetek USA), and cryo-sectioned at 10-µm thickness. For the analysis of the GFP intensity, cryosections were blocked in blocking buffer and incubated with mouse anti-GFP (1:1,000, Cell Signaling Technology) primary antibody overnight at 4°C, and then incubated with anti-mouse Alexa Fluor 488 secondary antibody (1:1,000; Invitrogen, Eugene, OR). Retinal nuclei were counterstained with DAPI and sections were mounted in Fluoro-Gel buffer (Electron Microscopy Sciences) for imaging. The images were generated and analyzed by the Nikon C2 confocal microscope (Nikon) with NIS-elements AR software, and further edited using Adobe Photoshop CS4, Version 11.0. The same imaging setting was used for analysis of all samples. For each eye, the retinal section with the strongest GFP signal was selected. Pixel intensity and area of GFP signals were measured along the entire length of the GFP-positive outer nuclear layer (ONL) using a method with ImageJ software as previously described (Jensen, E. C. (2013). Quantitative analysis of histological staining and fluorescence using ImageJ. Anat Rec (Hoboken). 296:378-81). GFP intensity was expressed and plotted as pixel/area (mean ± SEM) for each group (N=11 for each group). To analyze GFP distribution in retinas, we used micrographs of representative retinal sections without immunostaining showing average intensity Z projection of original GFP signals and plotted GFP intensity against distance from RPE using the Plot Profile function in ImageJ.
Histology: For retinal histologic analysis, cryosections were stained with hematoxylin and eosin (H and E) and photographed using a Nikon C2 confocal microscope with DS-Ri2 digital camera (Nikon). The thickness was evaluated by counting rows of nuclei across the ONL width at 200-µm intervals in the region between 200 and 1000 µm from the optic nerve head, in the inferior and superior halves of the retinal sections. In a single retinal section, the nuclei counts at each point in the defined regions were averaged to give an overall estimate of the ONL thickness for that retina. Nuclei were counted in ECVM-treated (n = 12) and untreated (n = 4) mice. For each group, two sections per sample were counted.
**Electroretinography (ERG):** Full-field Ganzfeld scotopic and photopic ERGs were recorded using an Espion E2 Electrophysiology System with a ColorDome Ganzfeld stimulus (Diagnosys LLC) after 12-hour dark adaptation. Animals were anesthetized, and pupils dilated. Measurements were made on 7 animals before and after ECVM application. Measurements before ECVM application served as the baseline. Responses were plotted as intensity-response functions for analysis. These ERG methods and analyses have been fully described previously (Song H., et al. (2014). Transgenic expression of constitutively active RAC1 disrupts mouse rod morphogenesis. Invest Ophthalmol Vis Sci. 55:2659-68; Marangoni D., et al. (2015). Intravitreal Ciliary Neurotrophic Factor Transiently Improves Cone-Mediated Function in a CNGB3-/- Mouse Model of Achromatopsia. Invest Ophthalmol Vis Sci. 56: 6810-22).
**Statistical Analysis:** Quantitative data are presented as mean ± SEM. Statistical comparisons of ERG a-wave and b-wave amplitudes and implicit time were done across a range of stimulus intensities using 2-way ANOVA and correcting for multiple comparisons using the Holm-Sidak method in GraphPad Prism 6.07 for Windows (GraphPad Software). To compare means between two groups, we first did a D'Agostino & Pearson omnibus normality test. For datasets that passed normality test, we used Student's t-test. If not, we used the Mann-Whitney test.

### Example 1

### Transduction Efficiency and Retinal Expression Following Ocular Delivery in Mice

Preliminary studies indicated that 10 µA direct current for 20 min did not adversely affect ocular structure or function (see **FIGS. 4A-4H** and **FIGS. 5A**, **5B**). We then evaluated whether this would enhance the retinal transduction efficiency of AAV8-CMV-GFP when applied immediately after 1x10⁹ vector genomes (vg) were injected into the vitreous of the mouse eye. Control eyes were injected with the GFP vector, but no current was applied. GFP expression was evaluated at 6 weeks post-injection (PI) by *in vivo* fundus imaging. The extent of the retinal area showing GFP expression (expressed in percent, **FIG. 1A**) was used to indicate efficiency of AAV8 vector transduction.

The mean GFP-expressed retinal area (30.5%) was significantly larger in eyes that were treated by the approach of electric-current vector mobility (ECVM, 30.5%) than for the control group (no current) (8.4%; p = 0.038; Mann Whitney test; **FIG.1****B**). Most control eyes (14 of 21, 67%) showed GFP expression across less than 10% of the central retina, and no control eyes showed more than 25% of the central retinal area transduced (**FIGS. 1A** **and** **1C**). By comparison, 7 of 16 (44%) injected eyes that received trans-ocular current showed more than 45% of the central retinal area transduced, and 4 of 16 (25%) eyes had GFP expression covering more than 65% of the central retina. These results indicate that ECVM significantly promotes the transduction efficiency of AAV8-CMV-GFP in WT mouse retina following intravitreal injection.

### Example 2

### Effects of Dose and Administration Time on

### Transduction Efficiency and Retinal Expression in Mice

We also evaluated the outcome with a smaller dose of 5x10⁸ vg/eye, and performed the analysis earlier after intravitreal injection, at 4.5 weeks (**FIGS. 2A-2D**). This increased the disparity between ECVM-treated and non-treated eyes. Of eyes without ECVM application, 82% (9 of 11) showed no GFP expression across the central retina (**FIGS. 2A** and **2B**). By comparison, almost half of eyes receiving ECVM had GFP expression across >20% of the central retina, and 2 of 11 treated eyes had >50% of the central retina transduced. The mean retinal area of GFP expression was significantly greater in the ECVM-treated eyes (22%) compared to injected controls that did not receive current (2.3%; p < 0.0001, Mann Whitney test; **FIG. 2B**). We noted that two eyes showed more expression than the remaining 9 eyes receiving ECVM treatment. Out of an abundance of caution, we re-ran the analysis after first removing these two samples, assuming they possibly had anomalously large retinal area transduced. The difference of ECVM-treated (n = 9) versus control (n = 11) eyes was still highly significant (p = 0.0003, Mann Whitney test; **FIG. 2C**).

### Example 3

### Evaluation of Gene Expression Intensity Following Ocular Delivery in Mice

We then collected the eyes in this lower dose group of 5x10⁸ vg/eye and compared GFP expression intensity by immunofluorescence on retinal histologic cryosections at 5 weeks after injection. Injected eyes receiving ECVM application showed about a 6-fold (5.9 ± 1.2) higher intensity compared to the control eyes (p = 0.0007; Mann Whitney test; **FIG. 2D**). These data further support the finding that trans-ocular ECVM increased the retinal transduction efficacy of AAV vectors following intravitreal administration.

### Example 4

### Evaluation of Gene Expression Distribution Following Ocular Delivery in Mice

We also evaluated the distribution of retinal cells transduced by the AAV8-EGFP in ECVM-treated eyes. GFP was observed in all retinal layers of ECVM-treated eyes (**FIGS. 3A-3E**). Most retinal sections showed greatest GFP fluorescence by deeper retinal cells, in the RPE and photoreceptors (**FIG. 3A**). Some sections showed transduction of ganglion cells in addition (**FIG. 3B**). This was confirmed by the retinal distribution profile of GFP intensity.

These studies demonstrate safe, efficient, and non-invasive methods to enhance gene transduction efficacy in the retina following intravitreal administration of gene expression vectors. We demonstrated that applying an electric micro-current across the eye *in vivo,* ECVM, with intravitreal vector dosing augments vector penetration of the internal limiting membrane (ILM) at the retinal surface and increases transduction of cells in the outer retina, without surgical removal or perforation of the ILM. The continuous 10 µA direct current for 20 min was safe for the adult mouse eye, and this approach demonstrates the potential for human application, as trans-ocular current has been used safely in other contexts.

### Example 5

### Evaluation of trans-ocular electrical micro-current enhancement of vector transduction in the rabbit retina

The effects of electric-current vector mobility (ECVM) on the penetration of an AAV-mediated vector across the internal limiting membrane (ILM) after intravitreal AAV vector administration, as demonstrated on mice (Examples 1-4, *supra*) were extended to a rabbit model. This study extended the experimental concept to rabbit as a second species, to evaluate whether trans-ocular electric current augments retinal transduction efficiency of AAV-CMV-EGFP administrated intravitreally by electric-current vector mobility (ECVM) in wild-type rabbits.

Adult wild-type New Zealand white rabbits, age 6-8 months old were used in these experiments. AAV-CMV-EGFP vector at 5E11 vector genome (vg) in 50 µl/eye were administered to the eye of adult WT rabbits by intravitreal injection. AAV serotype 8 was used for this demonstration. Immediately after intravitreal injection, a Burian-Allen ERG electrode was placed on the center of cornea with a thin layer of GONAK Hypromellose. A subdermal needle electrode was inserted subcutaneously behind the ipsilateral ear as the second electrode to complete the electrical circuit and create the condition of "whole eye stimulation." DC current (800 µA cornea-negative, monophasic, continuous) was applied for 20 min from a constant current, stimulus-isolator power supply (A365, World Precision Instruments, Sarasota, FL). Eyes injected with this same AAV-vector and dose, but without electric current application, served as controls.

Retinal AAV-GFP expression was evaluated by fundus imaging in vivo using a Spectralis fundus camera (Heidelberg Engineering). Eyes were harvested at the termination of the study, and retinal immunohistochemistry was performed to assess the distribution of retinal cells transduced by the AAV8-EGFP in current-treated and in no-current eyes.

We evaluated whether ECVM would enhance the retinal transduction efficiency of AAV-CMV-GFP when applied immediately after 5E11 vector genomes (vg) were injected into the vitreous of the rabbit eye. Control eyes were injected with the GFP vector, but no current was applied. Retinal GFP expression was monitored at 11 weeks post-injection (PI) by in vivo fundus autofluorescence imaging. The rabbit retina has a specialized region of myelinated nerve fibers radiating from the optical nervehead, termed the medullary rays (**FIG. 6A**). The fundus shows the majority of GFP signals in this area at 11 weeks after vector application. Thus, the extent of GFP expression in the medullated region was used to indicate efficiency of AAV-vector transduction. For the control eyes (**FIG. 6B**), the majority of cases showed no uptake and expression of the vector, as 13 of the 14 (93%) control eyes showed no or very little GFP signal across the medullated region; only 1 of the 14 (7%) had a visible but weak GFP signal across the medullary rays, which we designated as positive for GFP. By comparison, 6 of the 14 eyes (43%) receiving ECVM had positive GFP signals at the area of medullary rays (**FIG. 6C**).

We also noted that 6 of 14 (43%) of ECVM current-treated eyes showed peripheral retinal regions of punctate GFP expression, while this was observed in only 3 of 14 (21%) control eyes without ECVM application. Further, 1 of the 6 ECVM-treated eyes had a large area of punctate GFP signals in the inferior retina which was not observed for any control eye not receiving current.

We also evaluated the distribution of retinal cells transduced by the AAV-EFGP in ECVM-treated eyes. GFP was observed in several retinal layers, with the highest GFP intensities in the nerve fiber layer (i.e. the medullary rays). GFP was also seen in cells in the RGC layer, inner plexiform (IPL), inner nuclear (INL) and outer plexiform (OPL) layers. These regions were relatively smaller than we had seen for the ECVM method in wild type mice. However, it is important to recognize that the geometry of the mouse eye and rabbit eye are quite different in dimensions of the posterior surface of the lens relative to the retinal ILM surface, and we made no effort yet to optimize the electrical current nor the vector administration into the vitreous cavities in this rabbit experiment.

AAV transduction and expression was also prominent in some Muller glial cells, including processes stretching across the thickness of outer nuclear layer (ONL) to the outer limiting membrane (OLM). The other retinal regions showed abundant spotty GFP signals in the IPL. By comparison, control sections from eyes that did not receive trans-ocular current showed far less GFP expression and less extent of GFP signals across retina.

These data demonstrate that ECVM promotes the transduction efficiency of AAV-CMV-GFP in WT rabbit retina following intravitreal injection, and further demonstrates the utility of the ECVM technique to enhance penetration and expression of an AAV-vector construct into the retina of two species following administration into the vitreous.

### Example 6

### Evaluation of trans-ocular electrical micro-current enhancement of vector transduction in the rhesus monkey retina

The effects of electric-current vector mobility (ECVM) on the penetration of an AAV-mediated vector across the internal limiting membrane (ILM) after intravitreal AAV vector administration, as demonstrated on mice (Examples 1-4, *supra*) and rabbits (Example 5) were extended to a rhesus monkey model.

A rhesus monkey received intravitreal injection of AAV8-CMV-EGFP (1e12 vg/dose) on both eyes. One eye received ECVM (850 µA/20min) applied after intravitreal injection of the vector. The in vivo fundus fluorescence images (FIG. 7) show that injected-ECVM-treated eye (ECVM) had stronger GFP signal around and inside the fovea (arrow) compared with the fellow control eye. These data demonstrate that ECVM increases transduction efficiency of AAV vectors in nonhuman primate (rhesus monkey) retina following intravitreal vector delivery.

### Example 7

### Evaluation of vitreous distribution of AAV8 vector after application into the deep vitreous overlying the retina surface (para-retinal delivery) in rabbits

The vitreous is highly viscous which prevents rapid diffusion and distribution of gene therapy vectors throughout the vitreous. Thus, for a vector to reach the surface of the retina following intravitreal injection, it must be placed in close proximity to the retina. These studies were conducted to evaluate the partitioning and distribution of AAV8 vector in the posterior versus anterior vitreous 1 hour and 20 hours after deep intravitreal injection overlying the retina surface in rabbit eye.

Four New Zealand white rabbits were used in this experiment. Rabbits were sedated with ketamine (35 mg/kg) and xylazine (5 mg/kg), and pupils were dilated with phenylephrine 10% and tropicamide 1%. The injection site was treated with proparacaine 0.5% for topical anesthesia and 5% povidone iodine. AAV8-CMV 25 µl at dose of 1.5e11 vg/eye was mixed with a dye (1/100) and administered in the superotemporal quadrant using a 28 gauge, 0.5 inch long needle inserted 2.5 mm from the corneal limbus. When inserted through the pars plana, this needle extends close to the surface of the optic nerve. Injections were performed by inserting the needle under direct visualization using an operating microscope (Zeiss Lumera microscope equipped with the Alcon NGENUITY visualization system). For posterior injections, the needle tip was positioned just over the optic disc, bevel down, and vector solution was injected slowly, with pooling seen at the posterior pole. For anterior injections, the needle tip was inserted bevel up, approximately 2 mm inside the eye wall and vector solution was injected slowly at an angle away from the lens to avoid trauma. After injections, lubricating ointment (mineral oil 15%, white petrolatum 83%, lanolin 2%) was applied to each eye. While recovering from the anesthesia, rabbits #1 and #3 were placed on their right side and rabbits #2 and #4 on their left side. Rabbits #1 and #2 were euthanized at one hour; eyes were enucleated and snap frozen in liquid nitrogen (-186 °C) and kept at -80 °C until processed further. Rabbits #3 and #4 were returned to their cages for 20 hours and were mobile until they were euthanized and eyes enucleated.

The frozen vitreous was removed intact. The vitreous was then hemisected with a cold scalpel, and anterior and posterior segments were transferred into separate tubes which were kept in a -80°C freezer until qPCR was performed to determine the titers. Segments were thawed and 10 µl of vitreous humor from each was treated with DNase I in 50 µl reaction volume, with 50 µl of 200 mM EDTA added to inactivate DNase I enzyme. The reaction mix was diluted by transferring 10 µl into 990 µl of Q-PCR dilution buffer. It was digested for Q-PCR by adding 5 µl of DNase I. The plasmid pV5.2 CMV EGFP was digested with Sma I and used as to generate a standard curve to calculate the copy number of each vitreous samples.

To simplify the analysis, we calculated the vector concentration ratios, referenced to the location corresponding to the site of injection (posterior or anterior). At one-hour post administration, when the vector was delivered close to the retina, the viral copy number was substantially higher in the posterior vitreous than the anterior segment for the two eyes (13-fold to 34-fold higher than in the anterior segment). At one-hour after the injection into the anterior vitreous, the sample had 34-fold greater concentration in the anterior versus posterior vitreous samples. However, for one eye the reverse was seen. Hence in 3 of 4 eyes, the vector exhibited limited mixing during the first hour after administration.

The following table shows the data outcome of this study.

| ***Distribution of vector at 1 hour post-administration (vg*/*ml vitreous)*** | | | |
|---|---|---|---|
| Location of sample: | Posterior | Anterior Ratio (Injected location / Non-injected location) | |
| Posterior injection | 3.9 e10 | 0.3 e10 | 13 (Posterior: Anterior (P/A) ratio) |
| | 7.2 e10 | 0.21 e10 | 34 (Posterior: Anterior (P/A) ratio) |
| Anterior injection | .08 e10 | 2.7 e10 | 34 (Anterior: Posterior (A/P) ratio) |
| | 5.1 e10 | 0.4 e10 | 0.08 (Anterior: Posterior (A/P) ratio) |
| | | | (13-fold in opposite direction) |

| ***Distribution of vector at 20 hour post-administration*** | | | |
|---|---|---|---|
| Posterior injection | 3.2 e10 | 1.8 e10 | 1.8 (Posterior: Anterior (P/A) ratio) |
| | 0.5 e10 | 2.7 e10 | 0.2 (Posterior: Anterior (P/A) ratio) |
| | | | (5-fold in opposite direction) |
| Anterior injection | 1.6 e10 | 1.7 e10 | 1.1 (Anterior: Posterior (A/P) ratio) |
| | 8.0 e9 | 9.3 e9 | 1.2 (Anterior: Posterior (A/P) ratio) |

By 20 hours post-administration, the vector was more distributed between posterior and anterior segments, consistent with dispersion throughout the vitreous. In 3 of 4 eyes, the vector retained a trace higher concentration where it had been injected 20 hours earlier (1.1, 1.2, 1.8). One of 4 eyes showed mixing compared with 1 hour, but the concentration was 5-fold higher in the opposite direction, i.e. higher in the posterior vitreous despite having been injected anteriorly. Positioning of the rabbit after receiving the injection appeared not to affect the virus distribution.

These data demonstrate that positioning the needle tip into posterior vitreous near the retina for intravitreal injection results in high concentration of vector at the retina surface when sampled one hour after application. The concentration difference diminishes by 20 hours post-injection. The benefit of application deep into the vitreous is to obtain higher concentration of vector which improves entry into the retina with subsequent uptake by retinal cells and greater gene expression. This, in turn, leads to higher transduction efficiency and therapeutic benefit.

### Example 8

### Effects of Para-retinal delivery into the eyes of New Zealand rabbits

This study was conducted to further assess whether para-retinal gene delivery with an AAV8-CMV-EGFP vector provides a higher local concentration of vector in the posterior segment of the rabbit eye as compared to the anterior segment.

New Zealand white rabbits were administered the AAV8-CMV-EGFP vector in a para-retinal position (i.e., intravitreal injection immediately adjacent to the retinal surface as described in detail in Example 6) in this experiment. Rabbits were sedated as described in Example 7. AAV8-CMV-EGFP (30 µl) at dose of 1.5e11 vg/eye was mixed with a dye (1/100) and administered in the superotemporal quadrant using a 28 gauge, 0.5 inch long needle inserted 2.5 mm from the corneal limbus. When the needle is inserted through the pars plana, it extends close to the surface of the retina and optic nerve. Injections were performed by inserting the needle under direct visualization using an operating microscope (Zeiss Lumera microscope equipped with the Alcon NGENUITY visualization system). The visual observation allowed for immediate identification of "good" versus "failed" injections. This judgement was made immediately at the time of injection and before enucleation and any subsequent quantifications. Five eyes of 3 rabbits were deemed acceptable ("good") at the time of visualization (one of the eyes of one rabbit was not considered in this study because of an imperfect injection). The 6 mm distance between the posterior retina and lens in a rabbit eye is a difficult target to deliver well a para-retinal injection. For para-retinal injections, the needle tip was positioned just over the optic disc, bevel down, and vector solution was injected slowly, with pooling seen at the posterior pole. After injections, lubricating ointment (mineral oil 15%, white petrolatum 83%, lanolin 2%) was applied to each eye for comfort.

Enucleation was performed one hour after vector application. Each rabbit was euthanized by anesthetic overdose and eyes were removed by a standard enucleation method. Briefly, the eye globe was enucleated after separating the eye globe from the conjunctiva, third eyelid and extraocular muscles by scissors. Eyes were immersed in liquid nitrogen and transferred into a box filled with the dry ice.

Subsequently, the frozen vitreous was removed intact from the external ocular tissues. The vitreous was then hemisected and transferred into separate tubes which were kept in a -80 °C freezer until qPCR was performed to determine the titers (as described in Example 7). The segments were thawed and 10 µl of vitreous humor from each was treated with DNase I in a 50 µl reaction volume, with 50 µl of 200 mM EDTA added to inactivate DNase I enzyme. The reaction mix was diluted by transferring 10 µl into 990 µl of Q-PCR dilution buffer. Five microliters of the diluted sample was used for Q-PCR in a total reaction volume of 25 µl. The plasmid pV5.2 CMV EGFP was digested with Sma I and used to generate a standard curve to calculate the copy number of each vitreous samples.

Based on this study (n=5 rabbit eyes), 4 out of 5 eyes had higher vector genomes in the posterior portion of the vitreous adjacent to the retina than in the anterior vitreous adjacent to the lens. The Posterior/Anterior (P/A) segment ratio was calculated for each eye, and then the average P/A ratio for all eyes was calculated to be 7.43. In comparing the mean vector titer concentrations between the posterior and anterior segments for the 5 eyes, we found that after placing the vector para-retinally, the level of AAV vector genomes in the posterior segment was 3-fold higher than in the anterior segment of the rabbit eye one-hour post-injection (**FIG. 8**).

These results indicate that administering the large molecular AAV vector para-retinal (i.e. in the posterior portion of the vitreous and immediately adjacent to the retina surface) increases the vector quantity for at least the first hour after injection. This time period is important for the retina to take up the vector, as vector particles at a distance from the retina do not readily migrate to the retina for uptake, and these vector genomes are vulnerable for destruction via antibody and other immune mechanisms. These findings substantiate further the results in Example 7 demonstrating that para-retinal delivery provides a higher local concentration of viral genomes adjacent to the target retinal cells and bypasses the highly viscous vitreous body in the eye, and with this higher vector titer juxtaposed next to the retinal cells, there will be improved transduction and greater penetration of vector into retinal cells leading to greater benefit or efficacy at a given dose.

### Example 9

### Comparison of para-retinal and mid-vitreous delivery into the eye in non-human primates

To evaluate the potential improvement in retinal delivery of an expression vector in non-human primates, two monkeys were injected with an AAV8 vector (scAAV8-HRSP/IRBP-hRS/myc), bearing a human retinoschisin gene (RS1) that is myc-tagged at the same dose of 3e11 vector genomes (vg) per eye. One monkey received para-retinal delivery into both right (OD) and left (OS) eyes with viral vector. The second monkey received a typical mid-vitreous injection with viral vector into the right (OD) eye, and a mid-vitreous delivery of vehicle (negative control) into the contralateral eye or left (OS) eye. The monkey eyes were enucleated for evaluating the expression of the viral myc-tagged human retinoschisin (hRS1) 9.5 weeks post injection.

**FIG. 9A** shows the result of RS1 and myc tag antibody counterstaining in the fovea area (all cone photoreceptors) of injected eyes. The anti-RS1 antibody stains both endogenous monkey retinoschisin protein and the viral myc-tagged human RS1. The anti-myc tag antibody stains only the viral human RS1-myc tag fusion protein. **FIG. 9A** shows the distribution of immunofluorescence pattern in both eyes of monkey 1 that received a single para-retinal injection per eye. The top panel of **FIG. 9A** shows the retinal images of the right eye (OD), and the bottom panel shows the retinal images of the left eye (OS). Panels A and D show images of RS1 antibody staining. There is strong staining within the Inner Segment (IS) of cone photoreceptors (ONL), and staining in photoreceptor cells and outer plexiform layer (OPL), a layer of neuronal synapses in the retina) as well. Panels B and E show images of myc tag antibody staining. The myc tag antibody stained IS and photoreceptor cells detected in both injected eyes, and with strong staining in the OPL of the injected right eye. Panels C and F show the combined staining of anti-RS1 and anti-mycantibodies.

**FIG. 9B** shows the result of immunofluorescent assay in monkey eyes receiving standard mid-vitreous intravitreal delivery of either AAV8 vector (scAAV8-HRSP/IRBP-hRS/myc) or vehicle. The top panel of **FIG 9B** shows the images of the right eye (OD) which received a mid-vitreous injection of AAV vector into the eye, and the lower panel shows the images of the left eye (OS) that received a mid-vitreous injection of vehicle. Panels A and D show endogenous monkey RS1 staining in the IS, ONL, and OPL in both the right and left eye as observed in the first monkey. In contrast to the findings in monkey 1, with a mid-vitreous delivery of vector, there is little green fluorescent staining using the anti-myc tag antibody (**FIG 9B**, Panel B) indicating that the viral vector when introduced into the mid-vitreous portion of the eye results in poor transduction by the scAAV8-HRSP/IRBP-hRS/myc vector and consequently, low expression of myc-tagged human RS1. There is no myc tag staining found in the monkey eye injected with vehicle (**FIG 9B**, Panel E). Panels C and E are an overlay of anti-RS1 and anti-myc tag staining.

These data demonstrate that para-retinal delivery of an AAV vector containing the human RS1 cDNA and tagged with myc gave improved expression of the human RS1-myc tag fusion protein within the deep layers of the retina as compared to a regular mid-vitreous delivery of the same vector at the same dose. Moreover, the human RS1-myc tag expression scAAV8-HRSP/IRBP-hRS/myc was found in the inner segment of the photoreceptor, photoreceptor layer and outer plexiform layer in the fovea area of the retina, the intended target area.

While certain examples of ocular gene therapy treatments have been described with respect to mice, rabbits, and non-human primates, these examples have been presented by way of example only and are not intended to limit the scope of the present invention which is defined by the appended claims.

## Claims

1. A viral expression vector encoding a therapeutic protein for use in a method of treating an ophthalmic condition in a subject by intraocular injection of the viral expression vector and application of electric current to the eye.

2. The viral expression vector for use according to claim 1, wherein the ophthalmic condition is selected from the group consisting of Stargardt disease, X-linked retinoschisis, age-related macular degeneration (AMD), diabetic retinopathy, Leber congenital amaurosis (LCA), retinal detachment (due to disease, injury or spontaneous detachment), a cyst, a cystoid macular edema, retinitis pigmentosa, senile schisis, Fuchs corneal dystrophy, another corneal dystrophy, glaucoma, and a cataract.

3. The viral expression vector for use according to claim 1 or claim 2, wherein the intraocular injection comprises para-retinal injection of the viral expression vector between 0 and 13 mm from the surface of the retina in the posterior vitreous cavity of the eye; optionally between 0 and 3 mm from the surface of the retina in the posterior vitreous cavity of the eye.

4. The viral expression vector for use according to any one of claims 1-3, wherein:
(a) the electric current applied to the eye is between about 1.0 micro-ampere (µA) and about 15 milli-amperes (mA); and/or
(b) the electric current is applied to the eye for a period of between about 1 millisecond (ms) and about 24 hours.

5. The viral expression vector for use according to any one of claims 1-4, wherein the electric current applied to the eye:
(a) is applied as a fixed current; or
(b) is applied as a current selected from a variable current, a pulsed current, and a biphasic current.

6. The viral expression vector for use according to any one of claims 1-5, wherein the electric current is applied to the eye:
(a) concurrently with intraocular injection of the viral expression vector; or
(b) after intraocular injection of the viral expression vector.

7. The viral expression vector for use according to any one of claims 1-6, wherein the electric current is applied to the eye:
(a) up to 48 hours after intraocular injection of the viral expression vector;
(b) between 24 hours and 48 hours after intraocular injection of the viral expression vector;
(c) between 12 hours and 24 hours after intraocular injection of the viral expression vector;
(d) between 6 hours and 12 hours after intraocular injection of the viral expression vector;
(e) between 1 hour and 6 hours after intraocular injection of the viral expression vector; or
(f) between 1 minute and 60 minutes after intraocular injection of the viral expression vector.

8. The viral expression vector for use according to any one of claims 1-7, wherein the electric current is applied to the eye by contacting the subject with at least a first and a second electrode, wherein:
(a) the first electrode is placed in contact with the cornea of the eye of the subject and the second electrode is placed in contact with a separate portion of the eye of the subject; optionally wherein
(i) the second electrode is placed in contact with a portion of the eye outside of the cornea of the subject; and/or
(ii) the first electrode is placed in contact with a portion of the eye inside the eye or vitreous;
or
(b) both the first and the second electrodes are placed in contact with the eye of the subject and neither electrode is placed in contact with the cornea of the eye of the subject.

9. The viral expression vector for use according to any one of claims 1-8, wherein the viral expression vector is intraocularly injected into:
(a) the anterior segment of the eye by applying a positive voltage to the cornea, allowing the viral expression vector to enter at least one of the lens, iris, ciliary body, and cornea of the eye; or
(b) the posterior segment of the eye by applying a negative voltage to the cornea, thereby allowing the viral expression vector to enter the retina.

10. The viral expression vector for use according to any one of claims 1-9, wherein the expression vector is expressed in a cell of the eye selected from the group consisting of photoreceptors and retinal pigment epithelia (RPE), retinal ganglion cells, Muller cells, horizontal cells, bipolar cells, nerve fiber layer structure, and amacrine cells.

11. The viral expression vector for use according to any one of claims 1-10, wherein the viral expression vector comprises one or more nucleic acid sequences from a virus, an adenovirus, an adeno-associated virus (AAV), a retrovirus, vaccinia virus, herpes simplex virus, a lentivirus, a baculovirus, a recombinant virus that contains a nucleic acid molecule that is heterologous to its genome, a replication-defective virus, a virus that replicates in the nucleus of a cell in the subject, a virus that infects non-dividing cells or dividing cells, and a virus that exhibits tropism for tissues of the eye.

12. The viral expression vector for use according to any one of claims 1-11, wherein the viral expression vector comprises a viral genome.

13. The viral expression vector for use according to any one of claims 1-12, wherein the viral expression vector is an AAV expression vector.

14. The viral expression vector according to any one of claims 1-13, wherein the viral expression vector comprises adeno-associated virus inverted terminal repeats (ITRs).

15. The viral expression vector for use according to any one of claims 1-14, wherein:
(a) the viral expression vector comprises a promoter operably-linked to a nucleic acid sequence encoding a retinoschisin gene; optionally wherein:
(i) the nucleic acid sequence encoding the retinoschisin gene includes at least a 300 base pair portion of the first intron of the retinoschisin gene; or
(ii) the promoter is an eye-specific promoter; optionally wherein the promoter comprises a promoter selected from the group consisting of a CMV promotor, a retinoschisin promoter, a rhodopsin promoter, a rhodopsin kinase promoter, a CRX promoter, and any other promotor controlling expression in cells; and/or
(b) the viral expression vector is expressed in a cell of the eye selected from the group consisting of photoreceptors, retinal pigment epithelia (RPE), bipolar cells, Muller cells, and retinal ganglion cells; and/or
(c) the viral expression vector comprises an enhancer sequence that enhances the activity of the promoter; optionally wherein the enhancer sequence is an interphotoreceptor retinoid binding protein enhancer sequence; optionally wherein the interphotoreceptor retinoid-binding gene enhancer element replaces an Alu repeat sequence within the retinoschisin gene promoter; and/or
(d) the viral expression vector is flanked by adeno-associated virus inverted terminal repeats (ITRs); optionally wherein at least one of the ITRs has been modified at the rep nicking site or within the D region; optionally wherein:
(i) an ITR 5' to the expression cassette comprises a 15-base pair deletion of the ITR sequence in the A region of the palindrome of a wild type ITR sequence; optionally wherein the 5' ITR is further modified by removal of the D region which contains the rep nicking site in a wild type ITR sequence; or
(ii) an ITR 3' to the expression cassette is a full length, wild type ITR sequence; and/or
(e) the viral expression vector is a self-complementary adeno-associated virus (AAV) vector; and/or
(f) the viral expression vector comprises a human beta-globin 3' untranslated region and polyadenylation site.

## Patentansprüche

1. Ein viraler Expressionsvektor, der für ein therapeutisches Protein kodiert, zur Verwendung in einem Verfahren zur Behandlung einer Augenerkrankung bei einem Individuum durch intraokulare Injektion des viralen Expressionsvektors und Anlegen von elektrischem Strom an das Auge.

2. Viraler Expressionsvektor zur Verwendung nach Anspruch 1, wobei die Augenerkrankung aus der Gruppe ausgewählt ist, die aus Stargardt-Krankheit, X-chromosomaler Retinoschisis, altersbedingter Makuladegeneration (AMD), diabetischer Retinopathie, kongenitaler Leber-Amaurose (LCA), Netzhautablösung (aufgrund von Erkrankung, Verletzung oder spontaner Ablösung), einer Zyste, einem zystoiden Makulaödem, einer Retinitis pigmentosa, senilen Schisis, Fuchs-Hornhautdystrophie, einer anderen Hornhautdystrophie, einem Glaukom und einem Katarakt besteht.

3. Viraler Expressionsvektor zur Verwendung nach Anspruch 1 oder Anspruch 2, wobei die intraokulare Injektion eine pararetinale Injektion des viralen Expressionsvektors zwischen 0 und 13 mm von der Oberfläche der Netzhaut im hinteren Glaskörperraum des Auges, optional zwischen 0 und 3 mm von der Oberfläche der Netzhaut im hinteren Glaskörperraum des Auges, umfasst.

4. Viraler Expressionsvektor zur Verwendung nach einem der Ansprüche 1-3, wobei:
(a) der an das Auge angelegte elektrische Strom zwischen etwa 1,0 Mikroampere (µA) und etwa 15 Milliampere (mA) beträgt; und/oder
(b) der elektrische Strom für einen Zeitraum zwischen etwa 1 Millisekunde (ms) und etwa 24 Stunden an das Auge angelegt wird.

5. Viraler Expressionsvektor zur Verwendung nach einem der Ansprüche 1-4, wobei der an das Auge angelegte elektrische Strom:
(a) als Feststrom angelegt wird; oder
(b) als ein Strom angelegt wird, der aus einem variablen Strom, einem Impulsstrom und einem zweiphasischen Strom ausgewählt ist.

6. Viraler Expressionsvektor zur Verwendung nach einem der Ansprüche 1-5, wobei der elektrische Strom an das Auge angelegt wird:
(a) gleichzeitig mit der intraokularen Injektion des viralen Expressionsvektors; oder
(b) nach der intraokularen Injektion des viralen Expressionsvektors.

7. Viraler Expressionsvektor zur Verwendung nach einem der Ansprüche 1-6, wobei der elektrische Strom an das Auge angelegt wird:
(a) bis zu 48 Stunden nach der intraokularen Injektion des viralen Expressionsvektors;
(b) zwischen 24 Stunden und 48 Stunden nach der intraokularen Injektion des viralen Expressionsvektors;
(c) zwischen 12 Stunden und 24 Stunden nach der intraokularen Injektion des viralen Expressionsvektors;
(d) zwischen 6 Stunden und 12 Stunden nach der intraokularen Injektion des viralen Expressionsvektors;
(e) zwischen 1 Stunde und 6 Stunden nach der intraokularen Injektion des viralen Expressionsvektors; oder
(f) zwischen 1 Minute und 60 Minuten nach der intraokularen Injektion des viralen Expressionsvektors.

8. Viraler Expressionsvektor zur Verwendung nach einem der Ansprüche 1-7, wobei der elektrische Strom an das Auge angelegt wird, indem das Individuum mit mindestens einer ersten und einer zweiten Elektrode in Kontakt gebracht wird, wobei:
(a) die erste Elektrode mit der Hornhaut des Auges des Individuums in Kontakt gebracht wird und die zweite Elektrode mit einem separaten Anteil des Auges des Individuums in Kontakt gebracht wird; wobei optional
(i) die zweite Elektrode mit einem Anteil des Auges außerhalb der Hornhaut des Individuums in Kontakt gebracht wird; und/oder
(ii) die erste Elektrode mit einem Anteil des Auges innerhalb des Auges oder Glaskörpers in Kontakt gebracht wird;
oder
(b) sowohl die erste als auch die zweite Elektrode mit dem Auge des Individuums in Kontakt gebracht werden und keine der Elektroden mit der Hornhaut des Auges des Individuums in Kontakt gebracht wird.

9. Viraler Expressionsvektor zur Verwendung nach einem der Ansprüche 1-8, wobei der virale Expressionsvektor intraokular injiziert wird in:
(a) den vorderen Augenabschnitt durch Anlegen einer positiven Spannung an die Hornhaut, wodurch der virale Expressionsvektor in mindestens eines von Linse, Iris, Ziliarkörper und Hornhaut des Auges eintreten kann; oder
(b) den hinteren Augenabschnitt durch Anlegen einer negativen Spannung an die Hornhaut, wodurch der virale Expressionsvektor in die Netzhaut eintreten kann.

10. Viraler Expressionsvektor zur Verwendung nach einem der Ansprüche 1-9, wobei der Expressionsvektor in einer Zelle des Auges exprimiert wird, die aus der Gruppe ausgewählt ist, die aus Photorezeptoren und retinalem Pigmentepithel (RPE), retinalen Ganglienzellen, Müllerzellen, Horizontalzellen, Bipolarzellen, Nervenfaserschichtstruktur und amakrinen Zellen besteht.

11. Viraler Expressionsvektor zur Verwendung nach einem der Ansprüche 1-10, wobei der virale Expressionsvektor eine oder mehrere Nukleinsäuresequenzen von Folgendem umfasst: einem Virus, einem Adenovirus, einem adeno-assoziierten Virus (AAV), einem Retrovirus, Vacciniavirus, Herpes-simplex-Virus, einem Lentivirus, einem Baculovirus, einem rekombinanten Virus, das ein Nukleinsäuremolekül enthält, das heterolog zu seinem Genom ist, einem replikationsdefekten Virus, einem Virus, das im Nukleus einer Zelle in dem Individuum repliziert, einem Virus, das sich nicht teilende oder sich teilende Zellen infiziert, und einem Virus, das Tropismus für Gewebe des Auges zeigt.

12. Viraler Expressionsvektor zur Verwendung nach einem der Ansprüche 1-11, wobei der virale Expressionsvektor ein virales Genom umfasst.

13. Viraler Expressionsvektor zur Verwendung nach einem der Ansprüche 1-12, wobei der virale Expressionsvektor ein AAV-Expressionsvektor ist.

14. Viraler Expressionsvektor nach einem der Ansprüche 1-13, wobei der virale Expressionsvektor umgekehrte terminale Repetitionen (ITR) des adeno-assoziierten Virus umfasst.

15. Viraler Expressionsvektor zur Verwendung nach einem der Ansprüche 1-14, wobei:
(a) der virale Expressionsvektor einen Promotor umfasst, der betriebsfähig mit einer Nukleinsäuresequenz verknüpft ist, die für ein Retinoschisin-Gen kodiert; wobei optional:
(i) die Nukleinsäuresequenz, die für das Retinoschisin-Gen kodiert, mindestens einen 300-Basenpaar-Anteil des ersten Introns des Retinoschisin-Gens einschließt; oder
(ii) der Promotor ein augenspezifischer Promotor ist; wobei der Promotor optional einen Promotor umfasst, der aus der Gruppe ausgewählt ist, die aus einem CMV-Promotor, einem Retinoschisin-Promotor, einem Rhodopsin-Promotor, einem Rhodopsinkinase-Promotor, einem CRX-Promotor und einem anderen Promotor, der die Expression in Zellen steuert, besteht; und/oder
(b) der virale Expressionsvektor in einer Zelle des Auges exprimiert wird, die aus der Gruppe ausgewählt ist, die aus Photorezeptoren, retinalem Pigmentepithel (RPE), Bipolarzellen, Müllerzellen und retinalen Ganglienzellen besteht; und/oder
(c) der virale Expressionsvektor eine Enhancer-Sequenz umfasst, die die Aktivität des Promotors verstärkt; wobei die Enhancer-Sequenz optional eine Interphotorezeptor-Retinoidbindungsprotein-Enhancer-Sequenz ist; wobei das Interphotorezeptor-Retinoidbindungsgen-Enhancer-Element optional eine Alu-Repetitionssequenz innerhalb des Retinoschisin-Genpromotors ersetzt; und/oder
(d) der virale Expressionsvektor von umgekehrten terminalen Repetitionen (ITR) des adeno-assoziierten Virus flankiert wird; wobei mindestens eine von den ITR optional an der rep-Nicking-Stelle oder innerhalb der D-Region modifiziert worden ist; wobei optional:
(i) eine ITR 5' zu der Expressionskassette eine 15-Basenpaar-Deletion der ITR-Sequenz in der A-Region des Palindroms einer Wildtyp-ITR-Sequenz umfasst; wobei die 5'-ITR optional ferner durch Entfernen der D-Region, die die rep-Nicking-Stelle in einer Wildtyp-ITR-Sequenz enthält, modifiziert wird; oder
(ii) eine ITR 3' zu der Expressionskassette eine komplette Wildtyp-ITR-Sequenz ist; und/oder
(e) der virale Expressionsvektor ein selbstkomplementärer adeno-assoziierter Virus(AAV)-Vektor ist; und/oder
(f) der virale Expressionsvektor eine humane Beta-Globin-3'-untranslatierte Region und Polyadenylierungsstelle umfasst.

## Revendications

1. Vecteur d'expression viral codant pour une protéine thérapeutique destiné à être utilisé dans un procédé de traitement d'une affection ophtalmique chez un sujet par l'injection intraoculaire du vecteur d'expression viral et l'application d'un courant électrique à l'œil.

2. Vecteur d'expression viral destiné à être utilisé selon la revendication 1, dans lequel l'affection ophtalmique est choisie parmi le groupe constitué par la maladie de Stargardt, le rétinoschisis lié à l'X, la dégénérescence maculaire liée à l'âge (DMLA), la rétinopathie diabétique, l'amaurose congénitale de Leber (ACL), le décollement de la rétine (dû à une maladie, une blessure ou un décollement spontané), un kyste, un œdème maculaire cystoïde, la rétinite pigmentaire, le schisis sénile, la dystrophie cornéenne de Fuchs, une autre dystrophie cornéenne, le glaucome et une cataracte.

3. Vecteur d'expression viral destiné à être utilisé selon la revendication 1 ou la revendication 2, dans lequel l'injection intraoculaire comprend l'injection para-rétinienne du vecteur d'expression viral entre 0 et 13 mm de la surface de la rétine dans la cavité vitréenne postérieure de l'œil ; facultativement entre 0 et 3 mm de la surface de la rétine dans la cavité vitréenne postérieure de l'œil.

4. Vecteur d'expression viral destiné à être utilisé selon l'une quelconque des revendications 1 à 3, dans lequel :
(a) le courant électrique appliqué à l'œil est compris entre environ 1,0 microampère (µA) et environ 15 milliampères (mA) ; et/ou
(b) le courant électrique est appliqué à l'œil pendant une période comprise entre environ 1 milliseconde (ms) et environ 24 heures.

5. Vecteur d'expression viral destiné à être utilisé selon l'une quelconque des revendications 1 à 4, dans lequel le courant électrique appliqué à l'œil :
(a) est appliqué sous la forme d'un courant fixe ; ou
(b) est appliqué sous la forme d'un courant choisi parmi un courant variable, un courant pulsé, et un courant biphasique.

6. Vecteur d'expression viral destiné à être utilisé selon l'une quelconque des revendications 1 à 5, dans lequel le courant électrique est appliqué à l'œil :
(a) simultanément avec l'injection intraoculaire du vecteur d'expression viral ; ou
(b) après l'injection intraoculaire du vecteur d'expression viral.

7. Vecteur d'expression viral destiné à être utilisé selon l'une quelconque des revendications 1 à 6, dans lequel le courant électrique est appliqué à l'œil :
(a) jusqu'à 48 heures après l'injection intraoculaire du vecteur d'expression viral ;
(b) entre 24 heures et 48 heures après l'injection intraoculaire du vecteur d'expression viral ;
(c) entre 12 heures et 24 heures après l'injection intraoculaire du vecteur d'expression viral ;
(d) entre 6 heures et 12 heures après l'injection intraoculaire du vecteur d'expression viral ;
(e) entre 1 heure et 6 heures après l'injection intraoculaire du vecteur d'expression viral ; ou
(f) entre 1 minute et 60 minutes après l'injection intraoculaire du vecteur d'expression viral.

8. Vecteur d'expression viral destiné à être utilisé selon l'une quelconque des revendications 1 à 7, dans lequel le courant électrique est appliqué à l'œil en mettant le sujet en contact avec au moins une première et une deuxième électrodes, dans lequel :
(a) la première électrode est placée en contact avec la cornée de l'œil du sujet et la deuxième électrode est placée en contact avec une partie distincte de l'œil du sujet ; facultativement, dans lequel
(i) la deuxième électrode est placée en contact avec une partie de l'œil à l'extérieur de la cornée du sujet ; et/ou
(ii) la première électrode est placée en contact avec une partie de l'œil à l'intérieur de l'œil ou du vitré ;
ou
(b) la première et la deuxième électrodes sont toutes les deux placées en contact avec l'œil du sujet et aucune électrode n'est placée en contact avec la cornée de l'œil du sujet.

9. Vecteur d'expression viral destiné à être utilisé selon l'une quelconque des revendications 1 à 8, dans lequel le vecteur d'expression viral est injecté par voie intraoculaire dans :
(a) le segment antérieur de l'œil en appliquant une tension positive à la cornée, permettant au vecteur d'expression viral de pénétrer dans au moins un élément parmi le cristallin, l'iris, le corps ciliaire et la cornée de l'œil ; ou
(b) le segment postérieur de l'œil en appliquant une tension négative à la cornée, permettant ainsi au vecteur d'expression viral de pénétrer dans la rétine.

10. Vecteur d'expression viral destiné à être utilisé selon l'une quelconque des revendications 1 à 9, dans lequel le vecteur d'expression est exprimé dans une cellule de l'œil choisie parmi le groupe constitué par les photorécepteurs et les épithéliums pigmentaires de la rétine (EPR), les cellules ganglionnaires rétiniennes, les cellules de Müller, les cellules horizontales, les cellules bipolaires, la structure de couche de fibres nerveuses et les cellules amacrines.

11. Vecteur d'expression viral destiné à être utilisé selon l'une quelconque des revendications 1 à 10, dans lequel le vecteur d'expression viral comprend une ou plusieurs séquences d'acide nucléique provenant d'un virus, d'un adénovirus, d'un virus adéno-associé (VAA), d'un rétrovirus, du virus de la vaccine, du virus de l'herpès simplex, d'un lentivirus, d'un baculovirus, d'un virus recombinant qui contient une molécule d'acide nucléique qui est hétérologue à son génome, d'un virus à réplication défectueuse, d'un virus qui se réplique dans le noyau d'une cellule dans le sujet, d'un virus qui infecte des cellules qui ne se divisent pas ou des cellules qui se divisent, et d'un virus qui présente un tropisme pour les tissus de l'œil.

12. Vecteur d'expression viral destiné à être utilisé selon l'une quelconque des revendications 1 à 11, dans lequel le vecteur d'expression viral comprend un génome viral.

13. Vecteur d'expression viral destiné à être utilisé selon l'une quelconque des revendications 1 à 12, dans lequel le vecteur d'expression viral est un vecteur d'expression de VAA.

14. Vecteur d'expression viral selon l'une quelconque des revendications 1 à 13, dans lequel le vecteur d'expression viral comprend des répétitions terminales inversées (RTI) de virus adéno-associés.

15. Vecteur d'expression viral destiné à être utilisé selon l'une quelconque des revendications 1 à 14, dans lequel :
(a) le vecteur d'expression viral comprend un promoteur lié de manière fonctionnelle à une séquence d'acide nucléique codant pour un gène de la rétinoschisine ; facultativement dans lequel :
(i) la séquence d'acide nucléique codant pour le gène de la rétinoschisine inclut au moins une partie de 300 paires de bases du premier intron du gène de la rétinoschisine ; ou
(ii) le promoteur est un promoteur spécifique à l'œil ; facultativement dans lequel le promoteur comprend un promoteur choisi parmi le groupe constitué par un promoteur du CMV, un promoteur du rétinoschisine, un promoteur de la rhodopsine, un promoteur de la rhodopsine kinase, un promoteur du CRX, et tout autre promoteur contrôlant l'expression dans les cellules ; et/ou
(b) le vecteur d'expression viral est exprimé dans une cellule de l'œil choisie parmi le groupe constitué par des photorécepteurs, des épithéliums pigmentaires rétiniens (EPR), des cellules bipolaires, des cellules de Müller, et des cellules ganglionnaires rétiniennes ; et/ou
(c) le vecteur d'expression viral comprend une séquence activatrice qui active l'activité du promoteur ; facultativement, dans lequel la séquence activatrice est une séquence activatrice de la protéine liant les rétinoïdes aux interphotorécepteurs ; facultativement, dans lequel l'élément activateur du gène de liaison des rétinoïdes aux interphotorécepteurs remplace une séquence répétée Alu au sein du promoteur du gène de la rétinoschisine ; et/ou
(d) le vecteur d'expression viral est flanqué de répétitions terminales inversées (ITR) de virus adéno-associé ; facultativement, dans lequel au moins l'une des ITR a été modifiée au niveau du site de coupure rep ou au sein de la région D ; facultativement, dans lequel :
(i) une ITR en 5' de la cassette d'expression comprend une délétion de 15 paires de bases de la séquence d'ITR dans la région A du palindrome d'une séquence d'ITR de type sauvage ; facultativement, dans lequel l'ITR en 5' est modifiée en outre par l'élimination de la région D qui contient le site de coupure rep dans une séquence ITR de type sauvage ; ou
(ii) une ITR en 3' de la cassette d'expression est une séquence d'ITR de type sauvage, de pleine longueur ; et/ou
(e) le vecteur d'expression viral est un vecteur adéno-associé (VAA) auto-complémentaire ; et/ou
(f) le vecteur d'expression viral comprend une région 3' non traduite de la bêta-globine humaine et un site de polyadénylation.
